(19) Europäisches Patentamt / European Patent Office / Office européen des brevets

(11) **EP 4 174 967 A1**

(12) **EUROPEAN PATENT APPLICATION**
published in accordance with Art. 153(4) EPC

(43) Date of publication:
**03.05.2023 Bulletin 2023/18**

(21) Application number: **21829347.0**

(22) Date of filing: **09.06.2021**

(51) International Patent Classification (IPC):
$H01L\ 33/50^{(2010.01)}$    $A61B\ 1/00^{(2006.01)}$
$A61N\ 5/06^{(2006.01)}$    $A61N\ 5/067^{(2006.01)}$
$C09K\ 11/80^{(2006.01)}$    $H01L\ 33/00^{(2010.01)}$
$H01S\ 5/0225^{(2021.01)}$    $H01S\ 5/02251^{(2021.01)}$
$H01S\ 5/02255^{(2021.01)}$

(52) Cooperative Patent Classification (CPC):
A61B 1/00; A61N 5/06; C09K 11/77; H01L 33/00;
H01L 33/50; H01S 5/0225; H01S 5/02251;
H01S 5/02255

(86) International application number:
**PCT/JP2021/021910**

(87) International publication number:
**WO 2021/261251 (30.12.2021 Gazette 2021/52)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **24.06.2020 JP 2020108503**

(71) Applicant: **Panasonic Intellectual Property
Management Co., Ltd.
Osaka-shi, Osaka 540-6207 (JP)**

(72) Inventors:
• **NITTA, Mitsuru**
 **Osaka-shi, Osaka 540-6207 (JP)**
• **FUJIWARA, Chigusa**
 **Osaka-shi, Osaka 540-6207 (JP)**
• **OSHIO, Shozo**
 **Osaka-shi, Osaka 540-6207 (JP)**

(74) Representative: **Müller-Boré & Partner
Patentanwälte PartG mbB
Friedenheimer Brücke 21
80639 München (DE)**

(54) **LIGHT EMITTING DEVICE AND ELECTRONIC APPARATUS USING SAME**

(57) Provided is a light emitting device 1 including a light source 2 that emits primary light 6; and a wavelength converter 3 that includes a first phosphor 4 that absorbs the primary light 6 and emits first wavelength-converted light 7, wherein the light emitting device 1 emits output light 15 including the first wavelength-converted light 7, the first wavelength-converted light 7 is near-infrared light having a fluorescence intensity maximum value within a wavelength range of 700 nm or more and less than 800 nm, the first wavelength-converted light 7 mainly contains a broad fluorescent component 31 based on an electron energy transition of $^4T_2 \rightarrow {}^4A_2$ of $Cr^{34}$, and the broad fluorescent component 31 has a fluorescence spectrum half-width that is less than 100 nm.

FIG. 1

**Description**

TECHNICAL FIELD

The present disclosure relates to a light emitting device and an electronic apparatus using the same.

BACKGROUND ART

There is a known method to examine whether a tumor is present in living tissue by using optical properties of living tissue, such as absorption and scattering. Patent Literature 1 discloses a living tissue examination device including a light source that irradiates an examination area of living tissue with light and emits incoherent light, and a photodetector that detects reflected light or transmitted light. Such an examination device is generally called an optical coherence tomography device, and such a technique is called optical coherence tomography.

Meanwhile, there is also a known method called a fluorescence imaging method to examine whether a tumor is present in living tissue or to locate a tumor. In the fluorescence imaging method, a fluorescent agent is administered to a subject and is specifically accumulated in a tumor or the like in the subject, then the fluorescent agent is excited by light of a specific wavelength, and fluorescence emitted by the fluorescent agent is captured and displayed as a monitor image. In this way, by detecting the fluorescence emitted from the subject, it becomes possible to grasp the presence or absence and location of a tumor. Moreover, in the fluorescence imaging method, fluorescence emitted from a tumor part is detected, and thus it is possible to detect a tumor with greater accuracy than in optical coherence tomography.

The above-described fluorescence imaging method has recently attracted attention in the field of endoscopic medicine, especially an ICG fluorescence method using ICG (indocyanine green) as a fluorescent agent. ICG is excited by near-infrared light that is likely to penetrate a living body (for example, a peak wavelength of 770 nm), and emits near-infrared light having a longer-wavelength (for example, a fluorescence peak wavelength of 810 nm) than the former. Thus, by detecting the fluorescence emitted from ICG, it becomes possible to observe and treat a submucosal tumor and a lymph node. Note that light in the wavelength range of 650 nm or more and less than 1400 nm is likely to penetrate a living body because it is hardly scattered by hemoglobin and water in the living body. The above-described wavelength range is commonly referred to as a biological window.

In recent years, improvements in the excitation efficiency of ICG have been desired. Patent Literature 2 discloses a light emitting device including a conversion element containing a conversion material that converts a primary beam emitted from a semiconductor chip into a secondary beam having a wavelength of 700 to 2,000 nm. As near-infrared light emitted to ICG, one has been considered whose peak overlaps with the peak of the absorption spectrum of ICG.

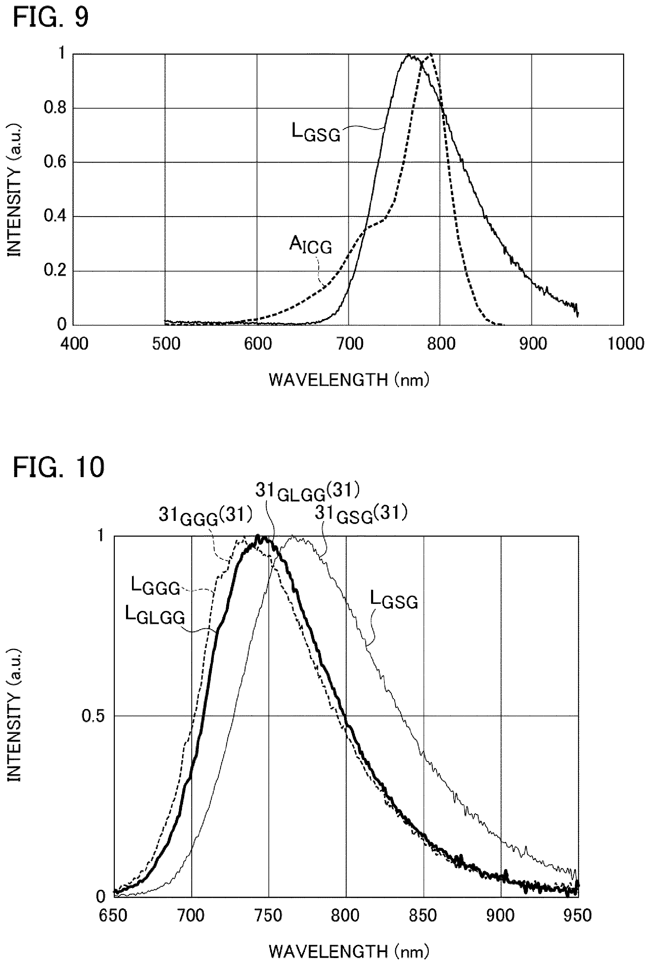

The absorption spectrum of ICG will be described. Fig. 9 illustrates an ICG absorption spectrum $A_{ICG}$, and a fluorescence spectrum $L_{GSG}$ of a conventional GSG-based phosphor, which are used in calculating the excitation efficiency of an ICG fluorescent agent. Note that in Fig. 9, the GSG-based phosphor means $(Gd_{0.75}La_{0.25})_3(Ga_{0.5}Sc_{0.47}Cr_{0.03})_2(GaO_4)_3$. As illustrated in Fig. 9, the peak wavelength of the ICG absorption spectrum is 790 nm. The peak wavelength of the fluorescence spectrum $L_{GSG}$ of the GSG-based phosphor is 765 nm.

CITATION LIST

PATENT LITERATURE

Patent Literature 1: Japanese Patent No. 5812461
Patent Literature 2: Japanese Unexamined Patent Application Publication No. 2018-518046

SUMMARY OF THE INVENTION

However, as in $A_{ICG}$ illustrated in Fig. 9, the absorption spectrum of ICG excited by near-infrared light does not have a symmetrical shape around the peak, and the half-width of the peak is relatively small. Thus, in a light emitting device that irradiates ICG with near-infrared light selected to simply overlap with the peak of the absorption spectrum of ICG, the area of overlap between the shape of a fluorescence spectrum of the near-infrared light and the shape of the absorption spectrum $A_{ICG}$ of ICG becomes small, and the excitation efficiency of ICG becomes low.

Note that the excitation efficiency of ICG (ICG excitation efficiency) when using the GSG-based phosphor illustrated in Fig. 9 means one calculated using the following equation (AI). In the following equation (AI), a "GSG emission spectrum intensity" means an "intensity of the fluorescence spectrum $L_{GSG}$ of the above-described GSG-based phosphor".

[Math 1]

$$\int_{500nm}^{800nm} [\text{GSG emission spectrum intensity}] \times [\text{ICG absorption spectrum intensity}]$$
$$= [\text{ICG excitation efficiency}] \quad (A1)$$

Note that the excitation efficiency of ICG when a phosphor X other than the GSG-based phosphor is used can be calculated by using an "X emission spectrum intensity", which is an "intensity of a fluorescence spectrum Lx of the phosphor X", instead of the "GSG emission spectrum intensity" in equation (AI). For example, when the phosphor X is a GLGG-based phosphor described below, a "GLGG emission spectrum intensity", which is an "intensity of a fluorescence spectrum $L_{GLGG}$ of the GLGG-based phosphor" is used instead of the "GSG emission spectrum intensity" in equation (AI).

The present disclosure has been made in consideration of these issues, which are inherent in the related art. An object of the present invention is to provide a light emitting device having high ICG excitation efficiency and an electronic apparatus using the light emitting device.

In response to the above issues, a light emitting device according to one aspect of the present disclosure includes a light source that emits primary light; and a wavelength converter that includes a first phosphor that absorbs the primary light and emits first wavelength-converted light, wherein the light emitting device emits output light including the first wavelength-converted light, the first wavelength-converted light is near-infrared light having a fluorescence intensity maximum value within a wavelength range of 700 nm or more and less than 800 nm, the first wavelength-converted light mainly contains a broad fluorescent component based on an electron energy transition of $^4T_2 \rightarrow ^4A_2$ of $Cr^{3+}$, and the broad fluorescent component has a fluorescence spectrum half-width that is less than 100 nm.

An electronic apparatus according to another aspect of the present disclosure includes the light emitting device.

BRIEF DESCRIPTION OF DRAWINGS

[FIG. 1] FIG. 1 is a schematic cross-sectional view of an example of a light emitting device according to a first embodiment.
[FIG. 2] FIG. 2 is a schematic cross-sectional view of an example of a light emitting device according to a second embodiment.
[FIG. 3] FIG. 3 is a schematic cross-sectional view of an example of a light emitting device according to a third embodiment.
[FIG. 4] FIG. 4 is a schematic cross-sectional view of an example of a light emitting device according to a fourth embodiment.
[FIG. 5] FIG. 5 is a schematic cross-sectional view of an example of a light emitting device according to a fifth embodiment.
[FIG. 6] FIG. 6 is a schematic cross-sectional view of an example of a light emitting device according to a sixth embodiment.
[FIG. 7] FIG. 7 is a schematic cross-sectional view of an example of a light emitting device according to a seventh embodiment.
[FIG. 8] FIG. 8 is a schematic cross-sectional view of an example of a light emitting device according to an eighth embodiment.
[FIG. 9] FIG. 9 is a diagram illustrating an absorption spectrum $A_{ICG}$ of ICG, and a fluorescence spectrum Lose of a conventional GSG-based phosphor, which are used to calculate the excitation efficiency of an ICG fluorescent agent.
[FIG. 10] FIG. 10 is a diagram illustrating a fluorescence spectrum of a first phosphor.
[FIG. 11] FIG. 11 is a diagram illustrating electron energy levels of $Cr^{3+}$.
[FIG. 12] FIG. 12 is a diagram illustrating a host crystal of the first phosphor.
[FIG. 13] FIG. 13 is a schematic diagram illustrating the configuration of an endoscope according to an embodiment.
[FIG. 14] FIG. 14 is a diagram schematically illustrating the configuration of an endoscope system according to an embodiment.

DESCRIPTION OF EMBODIMENTS

A light emitting device and an electronic apparatus according to an embodiment are described below with reference to the drawings. Note that dimensional ratios in the drawings are exaggerated for convenience of explanation and may differ from the actual ratios.

[Light emitting device]

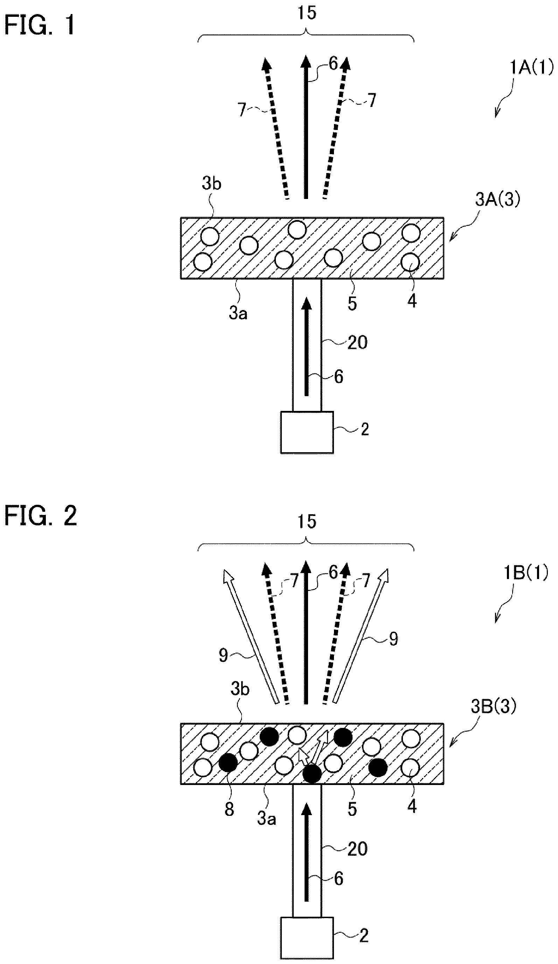

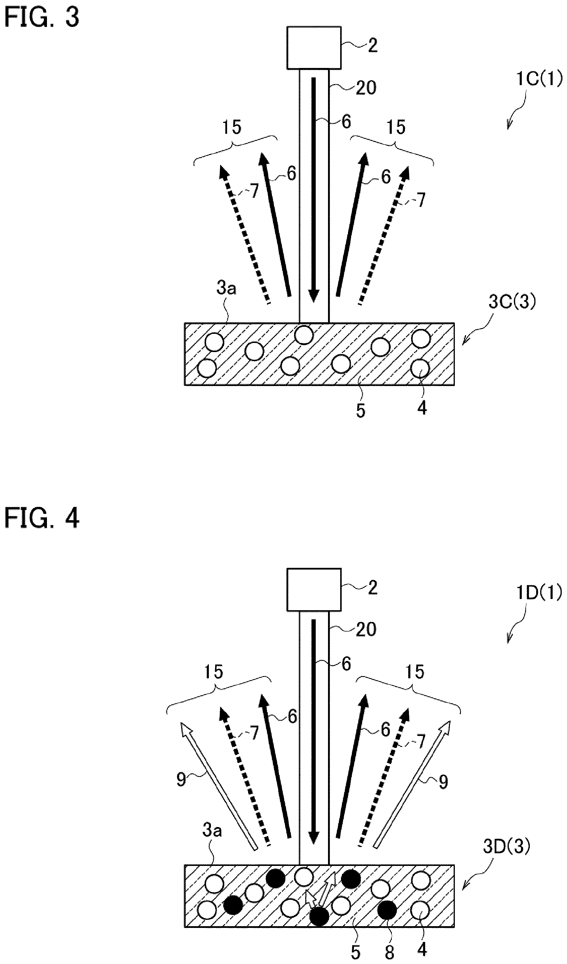

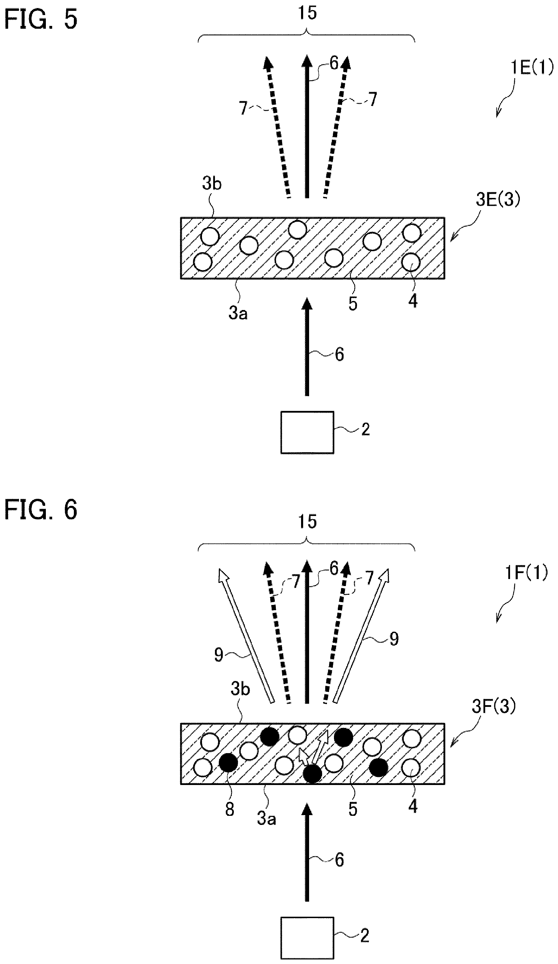

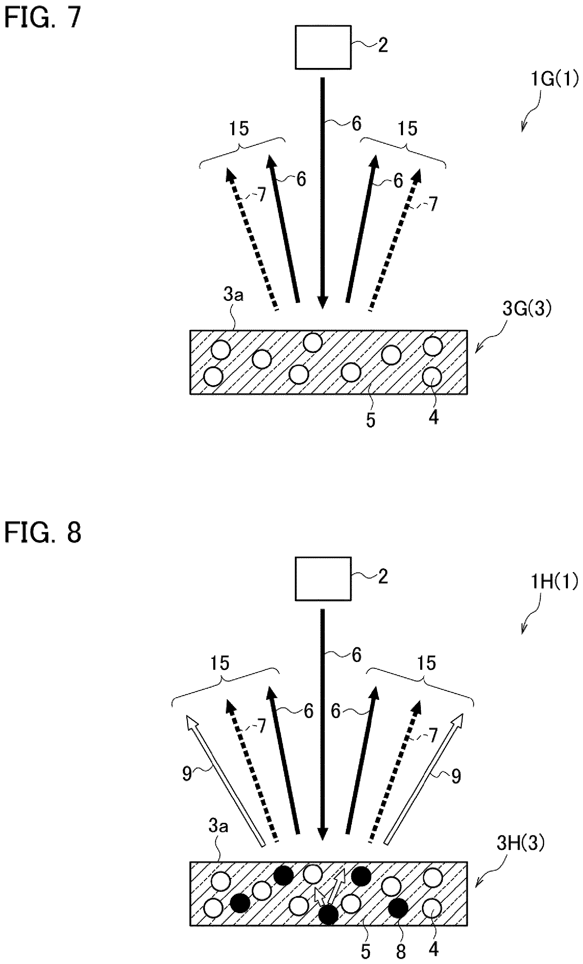

A light emitting device 1 (IA to 1H) according to an embodiment is illustrated in Figs 1 to 8. Fig. 1 is a schematic cross-sectional view of an example of a light emitting device according to a first embodiment. Fig. 2 is a schematic cross-sectional view of an example of a light emitting device according to a second embodiment. Fig. 3 is a schematic cross-sectional view of an example of a light emitting device according to a third embodiment. Fig. 4 is a schematic cross-sectional view of an example of a light emitting device according to a fourth embodiment. Fig. 5 is a schematic cross-sectional view of an example of a light emitting device according to a fifth embodiment. Fig. 6 is a schematic cross-sectional view of an example of a light emitting device according to a sixth embodiment. Fig. 7 is a schematic cross-sectional view of an example of a light emitting device according to a seventh embodiment. Fig. 8 is a schematic cross-sectional view of an example of a light emitting device according to an eighth embodiment.

Note that the light emitting devices 1A, 1B, 1C, and 1D according to the first to fourth embodiments are the light emitting devices 1E, 1F, 1G, and 1H according to the fifth to eighth embodiments, respectively, to which a light guide body 20 guiding primary light 6 is further added, and in which the remaining configuration is identical. That is, the light emitting devices 1E, 1F, 1G, and 1H according to the fifth to eighth embodiments are the light emitting devices 1A, 1B, 1C, and 1D according to the first to fourth embodiments, respectively, from which the light guide body 20 is removed.

The light emitting devices 1A to 1H according to the first to eighth embodiments include in common a light source 2 that emits the primary light 6, and a wavelength converter 3 including a first phosphor 4 that absorbs the primary light 6 to emit first wavelength-converted light 7. Thus, the light emitting devices 1A to 1H according to the first to eighth embodiments each emit output light 15 that includes the first wavelength-converted light 7.

In the light emitting devices 1A to 1H according to the first to eighth embodiments, when the primary light 6 emitted by the light source 2 enters the wavelength converter 3, a phosphor, such as the first phosphor 4 included in the wavelength converter 3, emits fluorescence. When receiving the primary light 6, the first phosphor 4 emits the first wavelength-converted light 7. The first wavelength-converted light 7 is near-infrared light having a fluorescence intensity maximum value within a wavelength range of 700 nm or more and less than 800 nm. The first wavelength-converted light 7 mainly contains a broad fluorescent component 31 based on an electron energy transition of $^4T_2 \rightarrow {}^4A_2$ of $Cr^{3+}$. The broad fluorescent component 31 has a fluorescence spectrum half-width of less than 100 nm. The above-described properties of the first wavelength-converted light 7 are described below as properties (A) to (C).

Note that the wavelength converter 3 (3A, 3B, 3E, and 3F) of the light emitting devices 1A, 1B, 1E, and 1F illustrated in Figs. 1, 2, 5, and 6 is configured to receive the primary light 6 at a front 3a and emit the first wavelength-converted light 7 from a back 3b. The wavelength converter 3 (3C, 3D, 3G, and 3H) of the light emitting devices 1C, 1D, 1G, and 1H illustrated in Figs. 3, 4, 7, and 8 is configured to receive the primary light 6 at the front 3a and emit the first wavelength-converted light 7 at the same front 3a.

Note that the wavelength converter 3 (3A to 3H) of the light emitting devices 1A to 1H according to the first to eighth embodiments receives the primary light 6, and emits the output light 15 containing at least the primary light 6 and the first wavelength-converted light 7.

[First embodiment]

The light emitting device 1A (1) according to the first embodiment will be described. The light emitting device 1A according to the first embodiment includes the light source 2, the wavelength converter 3 (3A), and the light guide body 20. In the light emitting device 1A, the light source 2 and the wavelength converter 3A are arranged spaced apart in such a way that the primary light 6 emitted by the light source 2 passes through the inside of the light guide body 20 to be emitted on the wavelength converter 3A.

In the light emitting device 1A, the light source 2 and the wavelength converter 3A are arranged spaced apart from each other, and thus it is possible to irradiate with a high degree of freedom an irradiation target, such as a human body, with the output light 15 emitted by the wavelength converter 3A and including the first wavelength-converted light 7. In the light emitting device 1A, the wavelength converter 3A is shaped and/or miniaturized to be suitable for an irradiation target, such as a human body, and thus it is possible to efficiently perform irradiation of the irradiation target.

(Light source)

The light source 2 emits the primary light 6. Light having a high emission intensity is used as the primary light 6. For example, laser light or synchrotron radiation from a high-output LED is used as the light having a high emission intensity. As the primary light 6, for example, light is used containing at least one of blue light having the maximum intensity of the spectral distribution within a wavelength range of 400 nm or more and less than 500 nm, or red light having the maximum intensity of the spectral distribution within a wavelength range of 600 nm or more and less than 660 nm. As the blue light, light having the maximum intensity of the spectral distribution within a wavelength range of 430 nm or more and less than 470 nm is preferably used. As the red light, light having the maximum intensity of the spectral distribution within a wavelength range of 610 nm or more and less than 650 nm is preferably used.

When the primary light 6 containing at least one of the above-described blue light or the above-described red light is used as the primary light 6, the primary light 6 is well absorbed by the first phosphor 4 activated with $Cr^{3+}$ and is efficiently wavelength-converted to the first wavelength-converted light 7. Thus, it is possible for the light emitting device 1A to emit output light having a large proportion of a fluorescent component based on the electron energy transition of $^4T_2 \rightarrow {}^4A_2$ of $Cr^{3+}$. The primary light 6 is preferably laser light because it is likely to obtain high-output light.

For example, a solid-state light emitting element is used as the light source 2. The light source 2 is preferably a solid-state light emitting element because of its excellent durability and long life. As the light source 2 that emits blue light, a solid-state light emitting element, such as a laser element and an LED that emit the above-described blue light, is used, for example. As the light source 2 that emits red light, a solid-state light emitting element, such as a laser element and an LED that emit the above-described red light, is used, for example.

Note that in the case of a blue laser element emitting blue light, it is easy to obtain a laser element having high efficiency and high output. Thus, it is preferable to use a blue laser element as the light source 2 to achieve high output of the light emitting device. A red laser element emitting red light has a small energy difference from a near-infrared light component and has a small energy loss associated with wavelength conversion. Thus, it is preferable to use a red laser element as the light source 2 to improve the efficiency of the light emitting device.

For example, a surface emitting laser diode is used as the light source 2. The light source 2 is a solid-state light emitting element having a rated light output of usually 1 W or more, preferably 3 W or more. When the rated light output of the light source 2 is within the above-described ranges, the primary light 6 having high output is emitted, and thus it is possible to achieve the high output of the light emitting device 1A.

Note that the upper limit of the rated light output is not limited. It is possible to achieve the high output of the light source 2 by making the light source 2 from multiple solid-state light emitting elements. However, for practicality, the rated light output of the light source 2 is usually less than 10 kW, preferably less than 3 kW.

The light density of the primary light 6 emitted on the first phosphor 4 is usually over 0.5 $W/mm^2$, preferably over 3 $W/mm^2$, more preferably over 10 $W/mm^2$, even more preferably over 30 $W/mm^2$. When the light density of the primary light 6 is within the above-described ranges, the first phosphor 4 is excited by the high density light, which enables the light emitting device 1A to emit a high-output fluorescent component. Note that if high-output LEDs exceeding 0.5 $W/mm^2$ are developed through the development of high-power LEDs in the future, a high-output LED is usable in the same way as the above-described laser.

(Wavelength converter)

The wavelength converter 3 (3A) includes the first phosphor 4 and a sealant 5. In the wavelength converter 3A, the first phosphor 4 is included in the sealant 5.

<First phosphor>

The first phosphor 4 is a phosphor that absorbs the primary light 6 and converts it into the first wavelength-converted light 7 having a wavelength longer than that of the primary light 6. The first phosphor 4 absorbs the primary light 6 and emits the first wavelength-converted light 7 containing fluorescence based on the electron energy transition of $^4T_2 \rightarrow {}^4A_2$ of $Cr^{3+}$. That is, the first wavelength-converted light 7 contains fluorescence based on the electron energy transition of $^4T_2 \rightarrow {}^4A_2$ of $Cr^{3+}$. Here, the fluorescence based on the electron energy transition of $^4T_2 \rightarrow {}^4A_2$ of $Cr^{3+}$ is fluorescence based on a spin-allowed transition of $Cr^{3+}$,

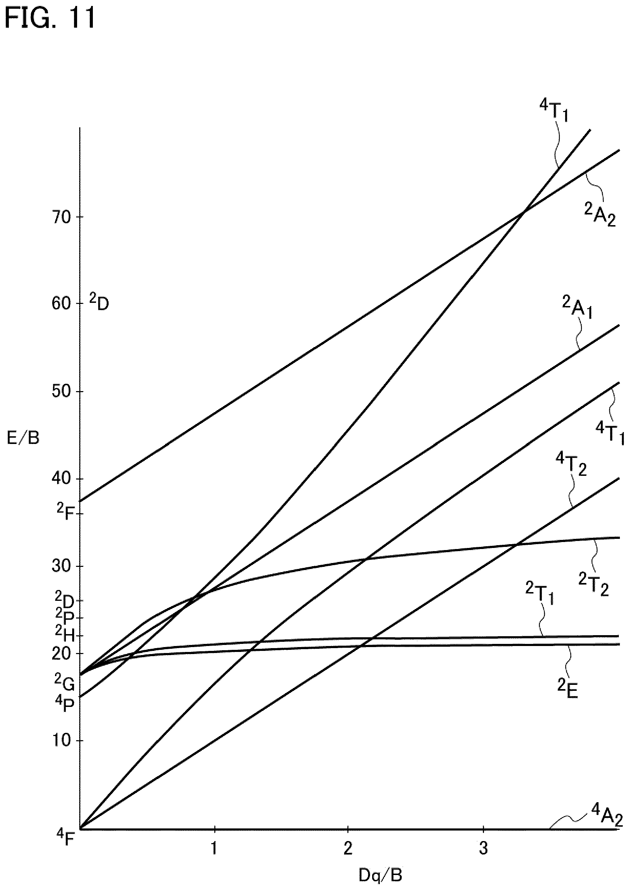

The electron energy transition of $^4T_2 \rightarrow {}^4A_2$ of $Cr^{3+}$ is described below. Fig. 11 illustrates electron energy levels of $Cr^{3+}$. Specifically, Fig. 11 is a Tanabe-Sugano diagram applied to six-coordinated $Cr^{3+}$, $Mn^{4+}$, and the like.

The horizontal axis in Fig. 11 illustrates Dq, which represents the magnitude of a ligand-field splitting, divided by B of the Laker parameter, which represents the strength of an electrostatic repulsive force acting between electrons. The horizontal axis in Fig. 11 can be understood as an indicator of the strength of the ligand field that $Cr^{3+}$ receives from surrounding ligands in a crystal. An example of ligands around $Cr^{3+}$ in a crystal is oxygen ions.

The vertical axis in Fig. 11 illustrates an energy E from a ground state divided by B of the above-described Laker parameter. The vertical axis in Fig. 11 can be understood as an indicator of the magnitude of an electron energy of an excited state formed by three 3d electrons forming the outermost electron cloud of $Cr^{3+}$, that is, an indicator indicating the energy difference between the excited state formed by the three 3d electrons and the ground state.

Fig. 11 illustrates that the electron energy of the excited state formed by 3d-orbit electrons of $Cr^{3+}$ in a phosphor

crystal takes several discrete states. Fig. 11 also illustrates that the state of the electron energy formed by electrons of $Cr^{3+}$ in a phosphor crystal changes depending on the type, number, and arrangement of surrounding ligands, the distance to ligands, and the like, and consequently, the energy difference between the excited state and the ground state changes. Moreover, Fig. 11 illustrates that the electron energies in the excited state, which take several discrete states, behave differently depending on ligand fields. Note that symbols, such as $^2E$, $^4T_2$, and $^4A_2$, illustrated in Fig. 11 are known symbols indicating each of the discrete electron energy states formed by the 3d-orbit three electrons of $Cr^{3+}$,

Here, the electron energy transition accompanied by fluorescence usually results in an electron energy transition from the lowest excited state ($^2T_1$ and $^2E$ or $^4T_2$ in Fig. 11) to the ground state ($^4A_2$ in Fig. 11). Thus, according to Fig. 11, when the strength of the ligand field received by $Cr^{3+}$ in a crystal is strong (the value on the horizontal axis in Fig. 11 is large), $Cr^{3+}$ exhibits fluorescence due to the electron energy transition from $^2T_1$ and $^2E$ to $^4A_2$. Fig. 11 also illustrates that when the strength of the ligand field is weak (the value on the horizontal axis in Fig. 11 is small), $Cr^{3+}$ exhibits fluorescence due to the electron energy transition from $^4T_2$ to $^4A_2$ (the electron energy transition of $^4T_2 \rightarrow {}^4A_2$). The first phosphor 4 exhibits fluorescence due to the latter electron energy transition of $^4T_2 \rightarrow {}^4A_2$.

Note that in the electron energy transition from $^2T_1$ and $^2E$ to $^4A_2$, as can be seen from Fig. 11, the fluorescence spectrum becomes linear because the energy difference does not change significantly even when the strength of the ligand field changes.

In contrast, in the electron energy transition from $^4T_2$ to $^4A_2$ (the electron energy transition of $^4T_2 \rightarrow {}^4A_2$), as can be seen from Fig. 11, when the strength of the ligand field changes, the energy difference changes in a significant manner, and thus the fluorescence spectrum takes a broad shape. The fluorescence spectrum of the first phosphor 4 has a broad shape because it is based on the electron energy transition (spin-allowed transition) of $^4T_2 \rightarrow {}^4A_2$.

Note that the energy transition between energy levels in the $^2T_1$ and $^2E$ to $^4A_2$ electron energy transition of 3d electrons of $Cr^{3+}$ is a parity-forbidden transition, and thus the fluorescence afterglow time is in a range of 100 $\mu$s or more and less than 50 ms. The afterglow time of the fluorescence based on this $Cr^{3+}$ is longer compared to that of $Ce^{3+}$ or $Eu^{2+}$ fluorescence (10 $\mu$s or less), which exhibit a parity-allowed transition. However, the electron energy transition from $^4T_2$ to $^4A_2$ of $Cr^{3+}$ (electron energy transition of $^4T_2 \rightarrow {}^4A_2$) is a spin-allowed transition between two states having the same spin, and thus the afterglow time is relatively short, around 100 $\mu$s.

Such a $Cr^{3+}$-activated phosphor that exhibits fluorescence due to a parity-forbidden (spin-allowed) electron energy transition exhibits a much longer afterglow property than an $Eu^{2+}$-activated phosphor that exhibits fluorescence due to a parity-allowed electron energy transition. The present disclosure is achieved by finding that the $Cr^{3+}$-activated phosphor exhibiting fluorescence due to a parity-forbidden electron energy transition has surprisingly little saturation of fluorescence output even though it exhibits a much longer afterglow property than an $Eu^{2+}$-activated phosphor.

The first wavelength-converted light 7 of the first phosphor 4 is fluorescence based on the spin-allowed type electron energy transition of $^4T_2 \rightarrow {}^4A_2$ of $Cr^{3+}$. Thus, the first phosphor 4 emits fluorescence that satisfies the following properties (A) to (C). In addition to the properties (A) to (C), the first phosphor 4 preferably emits the first wavelength-converted light 7 satisfying a property (D). Moreover, the first phosphor 4 may emit the first wavelength-converted light 7 satisfying a property (E) in addition to the properties (A) to (D).

[Property (A)]

The property (A) is that the first wavelength-converted light 7 is near-infrared light having a fluorescence intensity maximum value within a wavelength range of 700 nm or more and less than 800 nm. Here, the fluorescence intensity maximum value means the maximum fluorescence intensity at a peak where the fluorescence intensity exhibits the maximum value between peaks in a fluorescence spectrum. The first wavelength-converted light 7 is preferably near-infrared light having a fluorescence intensity maximum value within a wavelength range of 730 nm or more and less than 790 nm.

With a light emitting device satisfying the property (A), in which the first wavelength-converted light 7 is near-infrared light having a fluorescence intensity maximum value within a wavelength range of 700 nm or more and less than 800 nm, it is possible to easily obtain a point light source containing a large amount of a near-infrared component.

The light emitting device satisfying the property (A) is suitable as a medical or cosmetic light emitting device because the fluorescence spectrum of the first wavelength-converted light 7 is near-infrared light having a fluorescence intensity maximum value within a wavelength range of 700 nm or more and less than 800 nm, which is a suitable wavelength range for medical or cosmetic use.

[Property (B)]

The property (B) is that the first wavelength-converted light 7 mainly contains a broad fluorescent component 31 based on the electron energy transition of $^4T_2 \rightarrow {}^4A_2$ of $Cr^{3+}$.

Here, the broad fluorescent component 31 means a fluorescent component containing a "main broad peak" that is the highest peak in the fluorescence spectrum of the first wavelength-converted light 7 and has a fluorescence spectrum half-width of 50 nm or more. Hereinafter, a "fluorescent component containing a main broad peak" is also referred to as a "main broad peak fluorescent component".

The "fluorescence spectrum half-width" means the width (nm) of a wavelength between two points on the fluorescence spectrum that exhibit half the fluorescence intensity of the peak of the fluorescence spectrum. The fluorescence spectrum half-width is described using Fig. 10. Three fluorescence spectra $L_{GGG}$, $L_{GLGG}$, and $L_{GSG}$ illustrated in Fig. 10 are examples of fluorescence spectra of phosphors whose host crystals are $Gd_3Ga_2(GaO_4)_3$, $(Gd_{0.8}La_{0.2})_3Ga_2(GaO_4)_3$, and $(Gd_{0.75}La_{0.25})_3(Ga_{0.5}Sc_{0.5})_2(GaO_4)_3$, respectively.

One peak is in each of the three fluorescence spectra $L_{GGG}$, $L_{GLGG}$, and $L_{GSG}$ in Fig. 10. Positions of the peaks in the fluorescence spectra $L_{GGG}$, $L_{GLGG}$, and $L_{GSG}$ are at 735 nm, 745 nm, and 765 nm, respectively, and the fluorescence intensity of the peaks is 1.0, respectively. Thus, in each of the fluorescence spectra $L_{GGG}$, $L_{GLGG}$, and $L_{GSG}$, the fluorescence spectrum half-width is obtained by measuring the wavelength width (nm) between two points on each fluorescence spectrum at the fluorescence intensity of 0.5.

In the fluorescence spectra $L_{GGG}$, $L_{GLGG}$, and $L_{GSG}$ illustrated in Fig. 10, the half-widths of the fluorescence spectra are 92 nm, 91 nm, and 109 nm, respectively, which are 50 nm or more. Thus, each of the peaks in the fluorescence spectra $L_{GGG}$, $L_{GLGG}$, and $L_{GSG}$ is the "main broad peak".

Note that when the fluorescence spectrum of the first wavelength-converted light 7 contains only the main broad peak, as in the fluorescence spectra $L_{GGG}$, $L_{GLGG}$, and $L_{GSG}$ illustrated in Fig. 10, the above-described fluorescence spectrum is made from only the "main broad peak fluorescent component". Here, the broad fluorescent component 31 is the entire fluorescence spectrum, which is the "main broad peak fluorescent component". In the fluorescence spectra $L_{GGG}$, $L_{GLGG}$, and $L_{GSG}$, the entire fluorescence spectrum is the broad fluorescent component 31. In Fig. 10, the broad fluorescent components 31 of the $L_{GGG}$, $L_{GLGG}$, and $L_{GSG}$ are illustrated with symbols $31_{GGG}$, $31_{GLGG}$, and $31_{GSG}$, respectively.

In contrast, when the fluorescence spectrum of the first wavelength-converted light 7 contains a "sub peak", which is another peak, in addition to the main broad peak, the above-described fluorescence spectrum is made from a "main broad peak fluorescent component", and a "sub peak fluorescent component" containing the "sub peak". Here, the boundary between the "main broad peak fluorescent component" and the "sub peak fluorescent component" of the sub peak adjacent to the main broad peak is a straight line indicating a wavelength at which the fluorescence intensity is lowest between the main broad peak and the sub peak adjacent to the main broad peak. The boundary between the "sub peak fluorescent components" is a straight line indicating a wavelength at which the fluorescence intensity is lowest between two adjacent sub peaks.

Thus, when the fluorescence spectrum of the first wavelength-converted light 7 contains one or more "sub peaks", which are other peaks, in addition to the main broad peak, the broad fluorescent component 31 is the main broad peak fluorescent component, which is the remainder of the entire fluorescence spectrum excluding one or more "sub peak fluorescent components".

"The first wavelength-converted light 7 mainly contains the above-described broad fluorescent component 31" means that the amount of light of the broad fluorescent component 31 is the largest in the first wavelength-converted light 7.

Note that the above-described broad fluorescent component 31 includes the property (C) that "the fluorescence spectrum half-width is less than 100 nm" as described below. Thus, the broad fluorescent component 31 satisfies both the property (B) of 50 nm or more, and the property (C) of less than 100 nm in the fluorescence spectrum half-width.

In a light emitting device that satisfies the properties (A) and (B), the first wavelength-converted light 7 includes near-infrared light having a broad peak within a wavelength range of 700 nm or more and less than 800 nm, which is a suitable wavelength range for medical or cosmetic use. The light emitting device that satisfies the properties (A) and (B) is suitable as a medical or cosmetic light emitting device because it outputs near-infrared light that is absorbable by ICG with high efficiency, and broad near-infrared light that is effective for photocosmetics.

[Property (C)]

The property (C) is that the broad fluorescent component 31 of the first wavelength-converted light 7 has a fluorescence spectrum half-width of less than 100 nm.

The broad fluorescent component 31 of the first wavelength-converted light 7 preferably has a fluorescence spectrum half-width of less than 90 nm.

A light emitting device that satisfies the properties (A), (B), and (C) is suitable as a medical or cosmetic light emitting device because the fluorescence spectrum of the first wavelength-converted light 7 outputs near-infrared light that is absorbable by ICG with very high efficiency, and broad near-infrared light that is effective for photocosmetics.

[Property (D)]

The property (D) is that the percentage of a fluorescence intensity at the wavelength of 800 nm with respect to the fluorescence intensity maximum value in the fluorescence spectrum of the first wavelength-converted light 7 is less than 60%. Hereinafter, the above-described percentage of the fluorescence intensity is also referred to as an "800-nm fluorescence intensity percentage". The 800-nm fluorescence intensity percentage is preferably less than 50%.

When the 800-nm fluorescence intensity percentage is within the above-described ranges, the first wavelength-converted light 7 contains a large amount of a fluorescent component in the near-infrared wavelength range (650 to 1000 nm) in which light easily penetrates a living body, which is called a "biological window". Thus, it is possible for a light emitting device satisfying the property (D) to increase the intensity of near-infrared light penetrating a living body.

[Property (E)]

The property (E) is that the 1/10 afterglow of the first wavelength-converted light 7 is less than 1 ms. Here, the 1/10 afterglow means a time $\tau_{1/10}$ required from the time exhibiting the maximum emission intensity to the time to reach the 1/10 intensity of the maximum emission intensity. The 1/10 afterglow is preferably 10 $\mu$s or more and less than 1 ms, more preferably 10 $\mu$s or more and less than 800 $\mu$s, further more preferably 10 $\mu$s or more and less than 400 $\mu$s, particularly preferably 10 $\mu$s or more and less than 350 $\mu$s, more particularly preferably 10 $\mu$s or more and less than 100 $\mu$s.

When the 1/10 afterglow falls within the above-described ranges, the output of the fluorescence emitted by the first phosphor 4 is unlikely to saturate, even when the light density of excitation light that excites the first phosphor 4 is high. It is thus possible for a light emitting device satisfying the property (E) to emit high-output near-infrared light with less saturation of the fluorescence output when laser light having high light density is emitted.

Note that the 1/10 afterglow of the first wavelength-converted light 7 becomes longer than that of a short-afterglow (less than 10 $\mu$s) fluorescence based on the parity-allowed transition of $Ce^{3+}$, $Eu^{2+}$, or the like. This is because the first wavelength-converted light 7 mainly contains the broad fluorescent component (31) based on the spin-allowed electron energy transition of $^4T_2 \rightarrow {}^4A_2$ of $Cr^{3+}$, which has a relatively long afterglow.

[Crystal structure]

The first phosphor 4 preferably has a $Cr^{3+}$-activated structure in the host crystal. Preferably, as the first phosphor 4, one with a host crystal having a crystal structure of garnet represented by the following general formula (1) is used.

$$A'_{3}B'_{2}(C'O_{4})_{3} \qquad (1)$$

In the above-described formula (1), A', B', and C' are atoms constituting the host crystal of the garnet crystal structure. Specifically, A' is an "A-site", and the structure containing the "A-site" is an A-site structure in which oxygen atoms 0 are dodecahedrally coordinated around an atom A'. B' is a "B-site", and the structure containing the "B-site" is a B-site structure in which oxygen atoms 0 are octahedrally coordinated around an atom B'. C' is a "C-site", and the structure containing the "C-site" is a C-site structure in which oxygen atoms 0 are tetrahedrally coordinated around an atom C'. Note that a host crystal 41 is an atom other than Cr, which is the activating element of the first phosphor 4, and is a crystal constituting the host of the first phosphor 4.

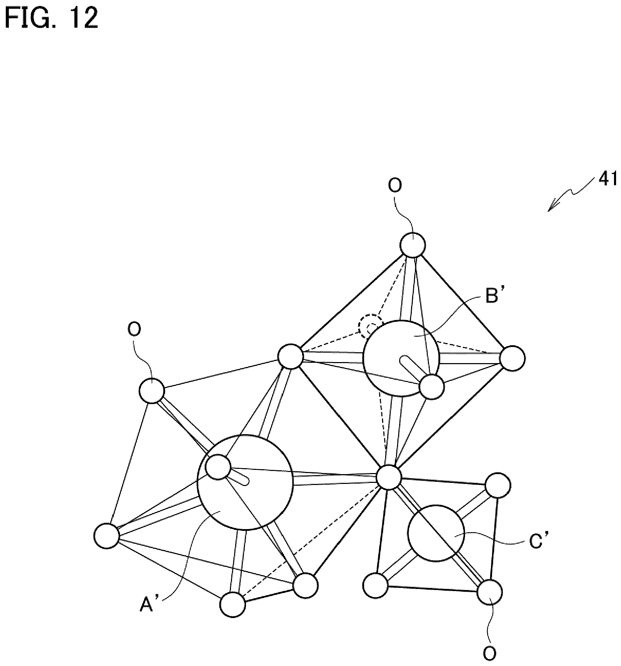

The host crystal 41 is described with reference to a drawing. Fig. 12 illustrates the host crystal 41 of the first phosphor 4. As illustrated in Fig. 12, the host crystal 41 has the A-site structure in which oxygen atoms 0 are dodecahedrally coordinated around the atom A', the B-site structure in which oxygen atoms 0 are octahedrally coordinated around the atom B', and the C-site structure in which oxygen atoms 0 are tetrahedrally coordinated around the atom C'.

In general formula (1), B' contains one element having an ion radius of 0.5 A or more and less than 0.8 A, for example. When B' contains one element having the above-described ion radius, the properties (B) and (C) are easily satisfied because the fluorescence spectrum half-width of the broad fluorescent component 31 of the first wavelength-converted light 7 becomes smaller, which is preferable. As B', for example, Al (ion radius 0.5 A), Ga (ion radius 0.62 A), or Sc (ion radius 0.81 Å) is used.

Note that Cr (ion radius 0.69 A), which is the activating element of the first phosphor 4, is usually replaced by part of B' in the host crystal. Thus, for example, when the host crystal is represented by $A'_{3}Ga_{2}(C'O_{4})_{3}$, the first phosphor 4 activated with Cr may be $A'_{3}(Ga, Cr)_{2}(C'O_{4})_{3}$. When the host crystal is represented by $A'_{3}(Ga, Sc)_{2}(C'O_{4})_{3}$, the first phosphor 4 activated with Cr may be $A'_{3}(Ga, Sc, Cr)_{2}(C'O_{4})_{3}$.

The host crystal 41 contains many A sites, many B sites, and many C sites. In the host crystal 41, each of these many A sites, many B sites, and many C sites are each in a particular arrangement. For example, many B sites in the host crystal 41 are arranged in a body-centered cubic lattice. That is, many B sites in the host crystal 41 are arranged to exist at a total of nine lattice points including eight lattice points positioned at apexes of the cube and one lattice point positioned at the center of gravity of the cube.

When the host crystal 41 of the first phosphor 4 is represented by the above-described general formula (1), examples of B', which is an atom to enter the B site, include Al, Ga, and Sc as described above. For example, when B' of the host crystal 41 is made from Ga, that is, only one kind, Ga is arranged in a B site at one lattice point positioned at the center of gravity of the cube, and Ga is arranged in B sites at the remaining eight lattice points positioned at the apexes of the cube.

Note that when B' of the host crystal 41 is made from Ga and Sc, which are two kinds, Ga or Sc is arranged in a B site at one lattice point positioned at the center of gravity of the cube, and one or more of Ga and Sc are arranged in B sites at the remaining eight lattice points positioned at the apexes of the cube.

Meanwhile, as described above, Cr that is the activating element of the first phosphor 4, is usually arranged by being substituted with part of B' of a B site making up the host crystal 41. Here, Cr is normally arranged not to enter an adjacent B site. That is, an atom, such as Al, Ga, or Sc, other than Cr is usually arranged between two Cr atoms arranged in a B site. For example, when Cr is arranged in a B site at one lattice point positioned at the center of gravity of the cube, usually, not Cr but Al, Ga, Sc, and the like are arranged in B sites at eight lattice points positioned at the remaining apexes of the cube. It is supposed that the reason why Cr is usually arranged not to enter the adjacent B site is that the activation amount of Cr in the first phosphor 4 is smaller than that of B', such as Al, Ga, and Sc.

$Cr^{3+}$ that is an activating element of the first phosphor 4, may be replaced with part of at least one of the atoms of A' or the atoms of C' in addition to B' in a B site. However, since Cr is an activating element and not an element constituting the host crystal 41, there is no problem when the first phosphor 4 is represented by $(A', Cr)_3B'_2(C'O_4)_3$, $A'_3B'_2((C', Cr)O_4)_3$, or the like.

In general formula (1), A' contains, for example, at least Gd (ion radius 1.05 A), and further contains La (ion radius 1.15 A) and/or Y (ion radius 0.90 A) as necessary. When A' contains Gd and La, A' is represented as (Gd, La). When A' contains Gd, it is easy to adjust the peak wavelength of the first phosphor 4 to 700 to 800 nm, which is preferable. When A' further contains La in addition to Gd, the peak wavelength of the first phosphor 4 becomes longer, and thus adjustment of the peak wavelength of the first phosphor 4 is possible in a more precise manner, which is preferable.

In general formula (1), C' usually contains only Ga (ion radius 0.62 A). As described above, Ga can be contained also as B' in a B site. Thus, in the host crystal 41 represented by general formula (1), Ga can be contained in one or both of B' in a B site and C' in a C site.

As the first phosphor 4, a phosphor is used in which the host crystal 41 represented as $A'_3B'_2(C'O_4)_3$, for example, is represented as $Gd_3Ga_2(GaO_4)_3$ (hereinafter also referred to as a "GGG host crystal"), $(Gd, La)_3Ga_2(GaO_4)_3$ (hereinafter also referred to as a "GLGG host crystal"), or the like, the phosphor being activated with Cr.

That is, a phosphor represented as $A'_3(B', Cr)_2(C'O_4)_3$, for example, and being $Gd_3(Ga, Cr)_2(GaO_4)_3$ (hereinafter also referred to as a "GGG phosphor") or $(Gd, La)_3(Ga, Cr)_2(GaO_4)_3$ (hereinafter also referred to as a "GLGG-based phosphor") is used as the first phosphor 4.

More specifically, as the GGG-based phosphor, a phosphor such as $Gd_3(Ga_{0.97}, Cr_{0.03})_2(GaO_4)_3$ (hereinafter also referred to as a "$GG_{0.97}G$ phosphor") is used.

More specifically, as the GLGG-based phosphor, a phosphor such as $(Gd_{0.8}La_{0.2})_3(Ga, Cr)_2(GaO_4)_3$ (hereinafter also referred to as a "$G_{0.8}L_{0.2}GG$ phosphor") or $(Gd_{0.8}La_{0.2})_3(Ga_{0.97}, Cr_{0.03})_2(GaO_4)_3$ (hereinafter also referred to as a "$G_{0.8}L_{0.2}G_{0.97}G$ phosphor") is used.

Note that as a phosphor having a structure similar to the GGG phosphor and the GLGG-based phosphor, a phosphor represented as $A'_3(B', Cr)_2(C'O4)_3$ and being $(Gd, La)_3(Ga, Sc, Cr)_2(GaO_4)_3$ (hereinafter also referred to as a "GSG-based phosphor") is known although it is not used as the first phosphor 4 because it does not satisfy the above-described property (C). More specifically, as the GSG-based phosphor, a phosphor of $(Gd_{0.75}La_{0.25})_3(Ga_{0.5}Sc_{0.47}Cr_{0.03})_2(GaO_4)_3$ (hereinafter also referred to as a "$G_{0.75}S_{0.47}G$ phosphor") is known.

[Crystal structure differences and properties]

The above-described GGG phosphor, $G_{0.8}L_{0.2}GG$ phosphor, and $G_{0.75}S_{0.47}G$ phosphor will be compared. There are differences in properties between the GGG phosphor, $G_{0.8}L_{0.2}GG$ phosphor, and $G_{0.75}S_{0.47}G$ phosphor.

Note that the $G_{0.8}L_{0.2}GG$ phosphor (GLGG-based phosphor) is obtained by changing an A site of the GGG phosphor from Gd to (Gd, La), such as $(Gd_{0.8}La_{0.2})$, through causing La, which has a larger ion radius than Gd, to be contained in Gd. The $G_{0.75}S_{0.47}G$ phosphor (GSG-based phosphor) is obtained by changing a B site of the $G_{0.8}L_{0.2}GG$ phosphor (GLGG-based phosphor) from (Ga, Cr) through causing Sc, which has a larger ion radius than Ga, to be contained in Ga. Specifically, the $G_{0.75}S_{0.47}G$ phosphor (GSG-based phosphor) is obtained by changing a B site of the $G_{0.8}L_{0.2}GG$ phosphor (GLGG-based phosphor) from (Ga, Cr) to (Ga, Sc, Cr), such as $(Ga_{0.5}Sc_{0.47}Cr_{0.03})$.

[Peak wavelength]

First, the peak wavelengths of fluorescence spectra will be compared. Note that the peak wavelengths of the fluorescence spectra of the GGG phosphor and the $G_{0.8}L_{0.2}GG$ phosphor are the peak wavelength of the broad fluorescent component 31 based on the electron energy transition of $^4T_2 \rightarrow {}^4A_2$ of $Cr^{3+}$ in the first wavelength-converted light 7.

The peak wavelengths of the GGG phosphor, the $G_{0.8}L_{0.2}GG$ phosphor, and the $G_{0.75}S_{0.47}G$ phosphor are 735 nm, 745 nm, and 765 nm, respectively.

It can be seen from these results that the peak wavelength becomes longer in the $G_{0.8}L_{0.2}GG$ phosphor (GLGG-based phosphor) obtained by mixing La having a larger ion radius than Gd into an A site of the GGG phosphor. It can be seen that the peak wavelength becomes longer in the $G_{0.75}S_{0.47}G$ phosphor (GSG-based phosphor) obtained by mixing Sc having a larger ion radius than Ga into a B site of the $G_{0.8}L_{0.2}GG$ phosphor (GLGG-based phosphor).

As described above, in a phosphor containing the host crystal 41 of general formula (1), it can be seen that the peak wavelength tends to become longer when an atom having a larger ion radius is made to be contained in an A site, a B site, or the like.

Thus, it is supposed that in a phosphor, such as the first phosphor 4, the fluorescence wavelength becomes larger as the inter-ligand distance between Cr-0 increases.

In a phosphor, such as the first phosphor 4, it is possible to adjust the inter-ligand distance between Cr-0 in a precise manner by introducing Gd, La, or the like to an A site or introducing Al, Ga, Sc, or the like to a B site, which is supposed to make it possible to broaden the fluorescence spectrum. Thus, with the first phosphor 4, it is thought that the broad fluorescent component 31 can be mainly contained by adjusting the composition.

[Fluorescence spectrum half-width]

Second, the half-widths of fluorescence spectra will be compared. Note that the fluorescence spectrum half-widths of the GGG phosphor and the $G_{0.8}L_{0.2}GG$ phosphor are the fluorescence spectrum half-width of the broad fluorescent component 31 based on the electron energy transition of $^4T_2 \rightarrow {}^4A_2$ of $Cr^{3+}$ in the first wavelength-converted light 7.

The half-widths of the fluorescence spectra of the GGG phosphor, $G_{0.8}L_{0.2}GG$ phosphor, and $G_{0.75}S_{0.47}G$ phosphor are 92 nm, 91 nm, and 109 nm, respectively.

These results indicate that the fluorescence spectrum half-width becomes larger in the $G_{0.75}S_{0.47}G$ phosphor (GSG-based phosphor) obtained by mixing Sc having a larger ion radius than Ga into a B site of the $G_{0.8}L_{0.2}GG$ phosphor (GLGG-based phosphor).

In other words, in a phosphor containing the host crystal 41 of general formula (1), it can be seen that the spectrum half-width tends to become larger when multiple kinds of atoms having different ion radii are made to be contained in a B-site.

Thus, it is supposed that in a phosphor, such as the first phosphor 4, the spectrum half-width decreases when the variation in the inter-ligand distance between Cr-0 is small.

In a phosphor, such as the first phosphor 4, it is possible to adjust the inter-ligand distance between Cr-0 in a precise manner by introducing Gd, La, or the like to an A site or introducing Al, Ga, Sc, or the like to a B site, which is supposed to make it possible to adjust the spectrum half-width in a precise manner. Thus, with the first phosphor 4, it is thought that the spectrum half-width can be adjusted in a precise manner by adjusting the composition.

The first phosphor 4 is preferably made from ceramic. When the first phosphor 4 is made from ceramic, the heat dissipation of the first phosphor 4 is enhanced and thus a decrease in the output of the first phosphor 4 due to temperature quenching is suppressed, and it becomes possible for the light emitting device to emit high-output near-infrared light.

In the light emitting device 1A, the first wavelength-converted light 7 emitted by the first phosphor 4 contains a specific fluorescent component that has high excitation efficiency of ICG and is based on the electron energy transition of $^4T_2 \rightarrow {}^4A_2$ of $Cr^{3+}$. Thus, it is possible for the light emitting device 1A to efficiently excite a fluorescent agent, such as ICG, and a light-sensitive agent (it is also a fluorescent agent), such as phthalocyanine.

The first wavelength-converted light 7 preferably has a light component over the entire wavelength range of 700 nm or more and less than 800 nm, more preferably over the entire wavelength range of 750 or more and less than 800 nm. This enables a fluorescent agent and a light-sensitive agent to absorb more efficiently the near-infrared light component emitted by the first phosphor 4, which makes it possible to increase the amount of near-infrared light emitted by the fluorescent agent or a heat ray emitted by the light-sensitive agent. Thus, when the first wavelength-converted light 7 has a light component over the entire wavelength range of 700 nm or more and less than 800 nm, the amount of near-infrared light emitted by the fluorescent agent and the heat ray emitted by the light-sensitive agent increases, and thus it is possible to obtain a light emitting device suitable for medical or cosmetic use.

Note that the fluorescence spectrum of the first wavelength-converted light 7 preferably does not include traces of a linear spectral component derived from the electron energy transition of $Cr^{3+}$. The linear spectral component derived from the electron energy transition of $Cr^{3+}$ is a long-afterglow fluorescent component due to the spin-forbidden transition of $Cr^{3+}$. When the fluorescence spectrum of the first wavelength-converted light 7 does not include the above-described traces, since the first wavelength-converted light 7 does not contain a long-afterglow fluorescent component due to the spin-forbidden transition of $Cr^{3+}$, it is possible to obtain a high-output point light source having less saturation of fluorescence output when irradiated with a laser light of high light density.

The wavelength converter 3 includes only the first phosphor 4 that contains fluorescence based on the electron energy transition of $Cr^{3+}$, as a phosphor. The first phosphor 4 contains no activator other than $Cr^{3+}$. Thus, light absorbed by the first phosphor 4 is converted into only the fluorescence based on the electron energy transition of $Cr^{3+}$. Thus, the light emitting device 1A in which the first phosphor 4 contains no activator other than $Cr^{3+}$ facilitates the design of output light to maximize the output proportion of the near-infrared fluorescent component.

It is preferable for the first phosphor 4 that the host crystal 41 have the crystal structure of garnet of the aforementioned general formula (1), as described above. The ease of compositional deformation of the garnet phosphor enables the preparation of numerous phosphor compounds. Thus, when the first phosphor 4 has a garnet crystal structure, it is easy to adjust a crystal field around $Cr^{3+}$ and to control the color tone of fluorescence based on the electron energy transition of $Cr^{3+}$.

Note that a phosphor having a garnet structure, especially an oxide, has a polyhedral particle shape close to a sphere and has excellent dispersibility in a phosphor particle group. Thus, when the first phosphor 4 has a garnet structure, it is relatively easy to manufacture the wavelength converter 3 having excellent optical transparency, which makes it possible to achieve high output of the obtained light emitting device 1A. Since a phosphor with a garnet crystal structure has a record of practical application as a phosphor for LEDs, the light emitting device 1A in which the first phosphor 4 has a garnet crystal structure is highly reliable.

As described above, the first phosphor 4 is preferably an oxide-based phosphor whose host crystal 41 is represented by the above-described general formula (1), more preferably an oxide phosphor. Note that the oxide-based phosphor refers to a phosphor containing oxygen but not nitrogen.

An oxide is a stable substance in the atmosphere, and thus when an oxide phosphor is heated through high density photoexcitation with laser light, the change in quality of a phosphor crystal due to oxidation in the atmosphere is less likely to occur compared to a nitride phosphor. When the first phosphor 4 is entirely an oxide-based phosphor, it is possible to obtain the light emitting device 1A having high reliability.

Note that the first phosphor 4 may contain two or more kinds of $Cr^{3+}$-activated phosphors. When the first phosphor 4 contains two or more kinds of $Cr^{3+}$-activated phosphors, it is possible to control at least the output light component in the near-infrared wavelength range. Thus, in a light emitting device in which the first phosphor 4 contains two or more kinds of $Cr^{3+}$-activated phosphors, it becomes easy to adjust the spectral distribution of the near-infrared fluorescent component.

<Sealant>

In the wavelength converter 3, the first phosphor 4 is contained in the sealant 5. Preferably, the first phosphor 4 is dispersed in the sealant 5. When the first phosphor 4 is dispersed in the sealant 5, it becomes possible to efficiently absorb the primary light 6 emitted by the light source 2 and efficiently convert the wavelength thereof to that of near-infrared light. When the first phosphor 4 is dispersed in the sealant 5, the wavelength converter 3 can be easily formed into a sheet or film shape.

The sealant 5 is made from at least one of an organic material or an inorganic material. The sealant 5 is preferably made from at least one of a transparent (translucent) organic material or a transparent (translucent) inorganic material. An example of the organic material sealant is a transparent organic material, such as a silicone resin. An example of the inorganic material sealant is a transparent inorganic material, such as low melting point glass.

Note that the wavelength converter 3 is preferably made from an inorganic material. An inorganic material here means a material other than an organic material, and includes ceramics and metals as a concept. When the wavelength converter 3 is made from an inorganic material, the thermal conductivity thereof is higher than that of the wavelength converter containing an organic material such as a sealing resin, which facilitates the design of heat dissipation. Thus, even when the first phosphor 4 is photoexcited at high density by the primary light 6 emitted by the light source 2, it is possible to suppress the temperature rise of the wavelength converter 3 in an effective manner. Consequently, the temperature quenching of the first phosphor 4 in the wavelength converter 3 is suppressed, which makes it possible to achieve high output of emitted light.

When the wavelength converter 3 is made from an inorganic material, the sealant 5 is preferably made from an inorganic material. The inorganic material for the sealant 5 is preferably zinc oxide (ZnO). When the sealant 5 is made from an inorganic material, the heat dissipation of the first phosphor 4 is further enhanced, and thus a decrease in output of the first phosphor 4 due to temperature quenching is suppressed, which makes it possible to emit high-output near-infrared light.

Note that as a modification of the light emitting device 1A, it is possible to replace the wavelength converter 3 with a wavelength converter that does not include the sealant 5. In this case, particles of the first phosphor 4 can be fixed to each other using an organic or inorganic binder. It is also possible to fix particles of the first phosphor 4 to each other through a heating reaction of the first phosphor 4. A generally used resin-based adhesive, ceramics fine particles, low-melting-point glass, or the like can be used as the binder. In the wavelength converter without the sealant 5, it is possible to reduce the thickness of the wavelength converter.

(Light guide body)

The light guide body 20 is a member arranged between the light source 2 and the wavelength converter 3A to guide the primary light 6 to the wavelength converter 3A. The primary light 6 is to pass through the inside of the light guide body 20.

In the light emitting device 1A, the light source 2 and the wavelength converter 3A are arranged spaced apart. The light guide body 20 allows the primary light 6 emitted by the light source 2 to pass through the inside of the light guide body 20 to be emitted on the wavelength converter 3A. In the light emitting device 1A, provision of the light guide body 20 enables the primary light 6 to be efficiently guided to the wavelength converter 3A even when the light source 2 and the wavelength converter 3A are arranged spaced apart.

For example, an optical fiber or the like is used as the light guide body 20.

(Action)

The action of the light emitting device 1A will be described. First, the primary light 6 emitted by the light source 2 passes through the inside of the light guide body 20 to be emitted on the front 3a of the wavelength converter 3A. The emitted primary light 6 penetrates the wavelength converter 3A. When the primary light 6 penetrates the wavelength converter 3A, the first phosphor 4 included in the wavelength converter 3A absorbs part of the primary light 6 and emits the first wavelength-converted light 7. In this way, the output light 15 including the primary light 6 and the first wavelength-converted light 7 is emitted from the back 3b of the wavelength converter 3A.

(Preferred use)

The light emitting device 1A emits the output light 15 including the first wavelength-converted light 7 that contains a large amount of a near-infrared fluorescent component based on the electron energy transition of $^4T_2 \rightarrow {}^4A_2$ of $Cr^{3+}$ and contains a specific fluorescent component having high ICG excitation efficiency. This makes the light emitting device 1A suitable as a medical or cosmetic near-infrared light source, or a sensing near-infrared light source.

The light emitting device 1A can be a medical or cosmetic light source, or a medical or cosmetic lighting device. The light emitting device 1A can be a lighting device for medical systems using, for example, a fluorescence imaging method or a photodynamic therapy. Note that these medical systems are those using fluorescent agents. Thus, the light emitting device 1A for the above-described medical systems can be also said to be a light emitting device for medical systems using fluorescent agents.

The light emitting device 1A is used as a light source or a lighting device that illuminates the interior of a living body with a broad near-infrared high-output light through a "biological window" and is capable of causing a fluorescent agent or a light-sensitive agent incorporated into the living body to fully function. Thus, it is possible to obtain a light emitting device with which a large therapeutic effect is expected, by using the light emitting device 1A as a medical or cosmetic light source, or as a medical or cosmetic lighting device, especially a lighting device for medical systems using a fluorescence imaging method or a photodynamic therapy.

The light emitting device 1A can also be a light source for a sensing device or a sensing system, or a lighting system for a sensing device or a sensing system. With the light emitting device 1A, it is possible to configure a high-sensitivity sensing device or a sensing system using an orthodox light-receiving element having a light reception sensitivity in the near-infrared wavelength range. Thus, with the light emitting device 1A as a light source for a sensing device or a sensing system, or as a lighting system for a sensing device or a sensing system, it is possible to obtain a light emitting device that facilitates the miniaturization of the sensing device or the sensing system and the broadening of the sensing range.

(Effect)

The first phosphor 4 included in the wavelength converter 3A of the light emitting device 1A emits the output light 15 including the first wavelength-converted light 7 having high ICG excitation efficiency. Thus, with the light emitting device 1A, it is possible to provide a light emitting device having high ICG excitation efficiency and to provide an electronic apparatus using the light emitting device.

Since the light emitting device 1A includes the light guide body 20, it is possible to have a configuration in which the light source 2 and the wavelength converter 3 are separated in distance. Thus, with the light emitting device 1A, the degree of freedom in designing the arrangement of the solid-state light emitting element 2 and the wavelength converter 3 in the light emitting device 1 becomes relatively large.

[Second embodiment]

The light emitting device 1B (1) according to the second embodiment will be described. The light emitting device 1B according to the second embodiment includes the light source 2, the wavelength converter 3 (3B), and the light guide body 20. The light emitting device 1B according to the second embodiment uses the wavelength converter 3B instead of the wavelength converter 3A of the light emitting device 1A according to the first embodiment. The light emitting device 1B according to the second embodiment differs from the light emitting device 1A according to the first embodiment only in the wavelength converter 3B. Thus, the wavelength converter 3B is described below, and for other members, the description of their configurations and actions is omitted or simplified.

(Wavelength converter)

The wavelength converter 3B includes the first phosphor 4, a second phosphor 8, and the sealant 5. In the wavelength converter 3B, the first phosphor 4 and the second phosphor 8 are included in the sealant 5. That is, the wavelength converter 3B of the light emitting device 1B further includes the second phosphor 8 that absorbs the primary light 6 and converts it into second wavelength-converted light 9 that has a wavelength longer than that of the primary light 6 and is different from the first wavelength-converted light 7.

The wavelength converter 3B is the same as the wavelength converter 3 of the light emitting device 1 according to the first embodiment except that it further includes the second phosphor 8. Thus, the second phosphor 8 is mainly described below, and the description of other configurations and actions is omitted or simplified.

<Second phosphor>

The second phosphor 8 is a phosphor that absorbs the primary light 6 and converts it into the second wavelength-converted light 9 that has a wavelength longer than that of the primary light 6 and is different from the first wavelength-converted light 7. In the light emitting device 1B, the wavelength converter 3B further includes the second phosphor 8 in addition to the first phosphor 4, and thus it is possible to emit white output light using additive mixing with the primary light 6 emitted by the light source 2, which is blue light, for example.

As described above, when the wavelength converter 3B further includes the second phosphor 8 in addition to the first phosphor 4, it becomes possible to control the shape of the fluorescence spectrum emitted by the wavelength converter 3B and the excitation properties. Thus, it is possible for the obtained light emitting device 1B to easily adjust the spectral distribution of the output light depending on use.

The second phosphor 8 included in the wavelength converter 3B is not limited as long as it can absorb the primary light 6 emitted by the light source 2 and emit the second wavelength-converted light 9 that is visible light. The second phosphor 8 is preferably a $Ce^{3+}$-activated phosphor having, as a host, a compound having, as a main component, at least one selected from the group of compounds consisting of garnet-type, calcium ferrite-type, and lanthanum silicon nitride ($La_3Si_6N_{11}$)-type crystal structures. The second phosphor 8 is preferably a $Ce^{3+}$-activated phosphor having, as a host, at least one compound selected from the group of compounds consisting of garnet-type, calcium-ferrite-type, and lanthanum silicon nitride ($La_3Si_6N_{11}$)-type crystal structures. When the above-described second phosphor 8 is used, it becomes possible to obtain output light having a large amount of light components from green based to yellow based light components.

As the second phosphor 8, for example, a $Ce^{3+}$-activated phosphor having, as a host, a compound (B) having, as a main component, at least one selected from the group consisting of $M_3RE_2(SiO_4)_3$, $RE_3Al_2(AlO_4)_3$, $MRE_2O_4$, and $RE_3Si_6N_{11}$ is used. As the second phosphor 8, for example, a $Ce^{3+}$-activated phosphor having, as a host, at least one selected from the group consisting of $M_3RE_2(SiO_4)_3$, $RE_3Al_2(AlO_4)_3$, $MRE_2O_4$, and $RE_3Si_6N_{11}$ is used. The second phosphor 8 is preferably a $Ce^{3+}$-activated phosphor having, as a host, a solid solution having the above-described compound (B) as an end component. Note that in the compound (B), M is an alkaline earth metal, and RE is a rare earth element.

The above-described second phosphor 8 well absorbs light within a wavelength range of 430 nm or more and 480 nm or less and converts it with high efficiency to green to yellow-based light having the intensity maximum within a wavelength range of 540 nm or more and less than 590 nm. Thus, by using the light source 2 that emits blue light as the primary light 6 and using the second phosphor 8, it is possible to easily obtain a visible light component.

When the wavelength converter 3B includes the first phosphor 4 and the second phosphor 8, the first phosphor 4 preferably emits the first wavelength-converted light 7 by absorbing at least one of the primary light 6 emitted by the light source 2 or the second wavelength-converted light 9 emitted by the second phosphor 8. As described above, the first phosphor 4 is preferably a phosphor that absorbs the primary light 6 emitted by the light source 2 and emits the first wavelength-converted light 7 that is near-infrared light.

The first phosphor 4 may be a phosphor that absorbs the second wavelength-converted light 9 emitted by the second phosphor 8 and emits the first wavelength-converted light 7 that is near-infrared light. That is, the second phosphor 8 may be excited by the primary light 6 to emit the second wavelength-converted light 9, and the first phosphor 4 may be excited by the second wavelength-converted light 9 to emit the first wavelength-converted light 7. Here, even when the first phosphor 4 is a phosphor hardly excited by the primary light 6, it becomes possible to excite the first phosphor 4 through the second phosphor 8 by using fluorescence emitted by the second phosphor 8.

Thus, when the first phosphor 4 absorbs the second wavelength-converted light 9 and emits the first wavelength-converted light 7, it becomes possible to select as the first phosphor 4 a phosphor that absorbs visible light, which expands the choice of the first phosphor 4 and facilitates the industrial production of the light emitting device 1B. When the first phosphor 4 absorbs the second wavelength-converted light 9 and emits the first wavelength-converted light 7, it becomes possible for the light emitting device 1B to emit the first wavelength-converted light 7 having a large near-infrared light component intensity.

Note that the second phosphor 8 may contain two or more kinds of $Cr^{3+}$-activated phosphors. When the second phosphor 8 contains two or more kinds of $Cr^{3+}$-activated phosphors, it is possible to control at least the output light component in the near-infrared wavelength range, which makes it easy to adjust the spectral distribution of the near-infrared fluorescent component.

(Action)

The action of the light emitting device 1B will be described. First, the primary light 6 emitted by the light source 2 passes through the inside of the light guide body 20 to be emitted on the front 3a of the wavelength converter 3B. The emitted primary light 6 penetrates the wavelength converter 3B. When the primary light 6 penetrates the wavelength converter 3B, the second phosphor 8 included in the wavelength converter 3B absorbs part of the primary light 6 and emits the second wavelength-converted light 9. Furthermore, the first phosphor 4 included in the wavelength converter 3B absorbs the primary light 6 and/or part of the second wavelength-converted light 9 to emit the first wavelength-converted light 7. In this way, the output light 15 including the primary light 6, the first wavelength-converted light 7, and the second wavelength-converted light 9 is emitted from the back surface 3b of the wavelength converter 3B.

(Preferred use)

The "preferred use" of the light emitting device 1B is the same as that of the light emitting device 1A, and thus the description is omitted.

(Effect)

The first phosphor 4 included in the wavelength converter 3B of the light emitting device 1B emits the output light 15 including the first wavelength-converted light 7 having high ICG excitation efficiency. Thus, with the light emitting device 1B, it is possible to provide a light emitting device having high ICG excitation efficiency and to provide an electronic apparatus using the light emitting device.

Since the light emitting device 1B includes the light guide body 20, it is possible to have a configuration in which the light source 2 and the wavelength converter 3 are separated in distance. Thus, with the light emitting device 1B, the degree of freedom in designing the arrangement of the solid-state light emitting element 2 and the wavelength converter 3 in the light emitting device 1 becomes relatively large.

[Third embodiment]

The light emitting device 1C (1) according to the third embodiment will be described. The light emitting device 1C according to the third embodiment includes the light source 2, the wavelength converter 3 (3C), and the light guide body 20. The light emitting device 1C according to the third embodiment uses the wavelength converter 3C instead of the wavelength converter 3A of the light emitting device 1A according to the first embodiment. The light emitting device 1C according to the third embodiment differs from the light emitting device 1A according to the first embodiment only in the wavelength converter 3C. Thus, the wavelength converter 3C is described below, and for other members, the description of their configurations and actions is omitted or simplified.

(Wavelength converter)

The wavelength converter 3C includes the first phosphor 4 and the sealant 5. In the wavelength converter 3C, the first phosphor 4 is included in the sealant 5. The wavelength converter 3C is the same as the wavelength converter 3A of the light emitting device 1 according to the first embodiment in that it includes the first phosphor 4 and the sealant 5, but the wavelength converter 3C has an optical effect different from that of the wavelength converter 3A.

In the wavelength converter 3A of the light emitting device 1A according to the first embodiment, the primary light 6 passing through the inside of the light guide body 20 to be emitted on the wavelength converter 3A penetrates the wavelength converter 3A. In contrast, in the wavelength converter 3C, most of the primary light 6 passing through the inside of the light guide body 20 to be emitted on the wavelength converter 3C enters the wavelength converter 3C from the front 3a of the wavelength converter 3C, and the remainder is reflected by the front 3a.

(Action)

The action of the light emitting device 1C will be described. First, the primary light 6 emitted by the light source 2 passes through the inside of the light guide body 20 to be emitted on the front 3a of the wavelength converter 3C. Most of the primary light 6 enters the wavelength converter 3C from the front 3a of the wavelength converter 3C, and the remainder is reflected by the front 3a. In the wavelength converter 3C, the first phosphor 4 excited by the primary light 6 emits the first wavelength-converted light 7, and the first wavelength-converted light 7 is emitted from the front 3a. In this way, the output light 15 including the primary light 6 and the first wavelength-converted light 7 is emitted from the front 3a of the wavelength converter 3C.

(Preferred use)

The "preferred use" of the light emitting device 1C is the same as that of the light emitting device 1A, and thus the description is omitted.

(Effect)

The first phosphor 4 included in the wavelength converter 3C of the light emitting device 1C emits the output light 15 including the first wavelength-converted light 7 having high ICG excitation efficiency. Thus, with the light emitting device 1C, it is possible to provide a light emitting device having high ICG excitation efficiency and to provide an electronic apparatus using the light emitting device.

Since the light emitting device 1C includes the light guide body 20, it is possible to have a configuration in which the light source 2 and the wavelength converter 3 are separated in distance. Thus, with the light emitting device 1C, the degree of freedom in designing the arrangement of the solid-state light emitting element 2 and the wavelength converter 3 in the light emitting device 1 becomes relatively large.

[Fourth embodiment]

The light emitting device 1D (1) according to the fourth embodiment will be described. The light emitting device 1D according to the fourth embodiment includes the light source 2, the wavelength converter 3 (3D), and the light guide body 20. The light emitting device 1D according to the fourth embodiment uses the wavelength converter 3D instead of the wavelength converter 3B of the light emitting device 1B according to the second embodiment. The light emitting device 1D according to the fourth embodiment differs from the light emitting device 1B according to the second embodiment only in the wavelength converter 3D. Thus, the wavelength converter 3D is described below, and for other members, the description of their configurations and actions is omitted or simplified.

(Wavelength converter)

The wavelength converter 3D includes the first phosphor 4, the second phosphor 8, and the sealant 5. In the wavelength converter 3D, the first phosphor 4 and the second phosphor 8 are included in the sealant 5. That is, the wavelength converter 3D of the light emitting device 1D further includes the second phosphor 8 that absorbs the primary light 6 and converts it into the second wavelength-converted light 9 that has a wavelength longer than that of the primary light 6 and is different from the first wavelength-converted light 7. The wavelength converter 3D is the same as the wavelength converter 3B of the light emitting device 1B according to the second embodiment in that it includes the first phosphor 4, the second phosphor 8, and the sealant 5, but the wavelength converter 3D has an optical action different from that of the wavelength converter 3B.

The second phosphor 8 used in the wavelength converter 3D is the same as that of the wavelength converter 3B of the light emitting device 1B according to the second embodiment, and thus the description is omitted. In the light emitting device 1D, the wavelength converter 3D includes the second phosphor 8, and thus it is possible to emit white output light using additive mixing with the primary light 6 emitted by the light source 2, which is blue light, for example.

As described above, when the first phosphor 4 and the second phosphor 8 are appropriately combined and used, it is possible to control the shape of the fluorescence spectrum of the first wavelength-converted light 7 and the excitation properties. Thus, it is possible for the obtained light emitting device C to easily adjust the spectral distribution of the output light depending on use.

In the wavelength converter 3B of the light emitting device 1B according to the second embodiment, the primary light 6 passing through the inside of the light guide body 20 to be emitted on the wavelength converter 3B penetrates the wavelength converter 3B. In contrast, in the wavelength converter 3D, most of the primary light 6 passing through the inside of the light guide body 20 to be emitted on the wavelength converter 3D enters the wavelength converter 3D from the front 3a of the wavelength converter 3D, and the remainder is reflected by the front 3a.

(Action)

The action of the light emitting device 1D illustrated in Fig. 4 will be described. First, the primary light 6 emitted by the light source 2 passes through the inside of the light guide body 20 to be emitted on the front 3a of the wavelength converter 3D. Most of the primary light 6 enters the wavelength converter 3D from the front 3a of the wavelength converter 3D, and the remainder is reflected by the front 3a. In the wavelength converter 3D, the second wavelength-converted light 9 is emitted by the second phosphor 8 excited by the primary light 6, and the first wavelength-converted light 7 is emitted by the first phosphor 4 excited by the primary light 6 and/or the second wavelength-converted light 9. Then, the first wavelength-converted light 7 and the second wavelength-converted light 9 are emitted from the front 3a. In this way, the output light 15 including the primary light 6, the first wavelength-converted light 7, and the second wavelength-converted light 9 is emitted from the front 3a of the wavelength converter 3D.

(Preferred use)

The "preferred use" of the light emitting device 1D is the same as that of the light emitting device 1A, and thus the description is omitted.

(Effect)

The first phosphor 4 included in the wavelength converter 3D of the light emitting device 1D emits the output light 15 including the first wavelength-converted light 7 having high ICG excitation efficiency. Thus, with the light emitting device 1D, it is possible to provide a light emitting device having high ICG excitation efficiency and to provide an electronic apparatus using the light emitting device.

Since the light emitting device 1D includes the light guide body 20, it is possible to have a configuration in which the light source 2 and the wavelength converter 3 are separated in distance. Thus, with the light emitting device 1D, the degree of freedom in designing the arrangement of the solid-state light emitting element 2 and the wavelength converter 3 in the light emitting device 1 becomes relatively large.

[Fifth embodiment]

The light emitting device 1E (1) according to the fifth embodiment will be described. The light emitting device 1E according to the fifth embodiment includes the light source 2 and the wavelength converter 3 (3E). The light emitting device 1E according to the fifth embodiment is the light emitting device 1A according to the first embodiment from which the light guide body 20 is removed. The light emitting device 1E according to the fifth embodiment differs from the light emitting device 1A according to the first embodiment only in the light guide body 20. Thus, the action of the light emitting device 1E not including the light guide body 20 is mainly described below, and for other members, the description of their configurations and actions is omitted or simplified.

(Action)

The action of the light emitting device 1E will be described. First, the primary light 6 emitted by the light source 2 is emitted on the front 3a of the wavelength converter 3E. The emitted primary light 6 penetrates the wavelength converter 3E. When the primary light 6 penetrates the wavelength converter 3E, the first phosphor 4 included in the wavelength converter 3E absorbs part of the primary light 6 to emit the first wavelength-converted light 7. In this way, the output light 15 including the primary light 6 and the first wavelength-converted light 7 is emitted from the back 3b of the wavelength converter 3E.

(Preferred use)

The "preferred use" of the light emitting device 1E is the same as that of the light emitting device 1A, and thus the description is omitted.

(Effect)

The first phosphor 4 included in the wavelength converter 3E of the light emitting device 1E emits the output light 15 including the first wavelength-converted light 7 having high ICG excitation efficiency. Thus, with the light emitting device 1E, it is possible to provide a light emitting device having high ICG excitation efficiency and to provide an electronic apparatus using the light emitting device.
Since the light guide body 20 is not included, the light emitting device 1E is inexpensive.

[Sixth embodiment]

The light emitting device IF (1) according to the sixth embodiment will be described. The light emitting device IF according to the sixth embodiment includes the light source 2 and the wavelength converter 3 (3F). The light emitting device IF according to the sixth embodiment is the light emitting device 1B according to the second embodiment from which the light guide body 20 is removed. The light emitting device IF according to the sixth embodiment differs from the light emitting device 1B according to the second embodiment only in the light guide body 20. Thus, the action of the light emitting device IF not including the light guide body 20 is mainly described below, and for other members, the description of their configurations and actions is omitted or simplified.

(Action)

The action of the light emitting device IF will be described. First, the primary light 6 emitted by the light source 2 is emitted on the front 3a of the wavelength converter 3F. The emitted primary light 6 penetrates the wavelength converter 3F. When the primary light 6 penetrates the wavelength converter 3F, the second phosphor 8 included in the wavelength converter 3F absorbs part of the primary light 6 to emit the second wavelength-converted light 9. Furthermore, the first phosphor 4 included in the wavelength converter 3F absorbs the primary light 6 and/or part of the second wavelength-converted light 9 to emit the first wavelength-converted light 7. In this way, the output light 15 including the primary light 6, the first wavelength-converted light 7, and the second wavelength-converted light 9 is emitted from the back 3b of the wavelength converter 3F.

(Preferred use)

The "preferred use" of the light emitting device IF is the same as that of the light emitting device 1A, and thus the description is omitted.

(Effect)

The first phosphor 4 included in the wavelength converter 3F of the light emitting device IF emits the output light 15 including the first wavelength-converted light 7 having high ICG excitation efficiency. Thus, with the light emitting device 1F, it is possible to provide a light emitting device having high ICG excitation efficiency and to provide an electronic apparatus using the light emitting device.
Since the light guide body 20 is not included, the light emitting device IF is inexpensive.

[Seventh embodiment]

The light emitting device 1G (1) according to the seventh embodiment will be described. The light emitting device 1G according to the seventh embodiment includes the light source 2 and the wavelength converter 3 (3G). The light emitting device 1G according to the seventh embodiment is the light emitting device 1C according to the third embodiment from which the light guide body 20 is removed. The light emitting device 1G according to the seventh embodiment differs from the light emitting device 1C according to the third embodiment only in the light guide body 20. Thus, the action of the light emitting device 1G not including the light guide body 20 is mainly described below, and for other members, the description of the configurations and actions is omitted or simplified.

(Action)

The action of the light emitting device 1G will be described. First, the primary light 6 emitted by the light source 2 is emitted on the front 3a of the wavelength converter 3G. Most of the primary light 6 enters the wavelength converter 3G from the front 3a of the wavelength converter 3G, and the remainder is reflected by the front 3a. In the wavelength converter 3G, the first phosphor 4 excited by the primary light 6 emits the first wavelength-converted light 7, and the first wavelength-converted light 7 is emitted from the front 3a. In this way, the output light 15 including the primary light 6 and the first wavelength-converted light 7 is emitted from the front 3a of the wavelength converter 3G.

(Preferred use)

The "preferred use" of the light emitting device 1G is the same as that of the light emitting device 1A, and thus the description is omitted.

(Effect)

The first phosphor 4 included in the wavelength converter 3G of the light emitting device 1G emits the output light 15 including the first wavelength-converted light 7 having high ICG excitation efficiency. Thus, with the light emitting device 1G, it is possible to provide a light emitting device having high ICG excitation efficiency and to provide an electronic apparatus using the light emitting device.

Since the light guide body 20 is not included, the light emitting device 1G is inexpensive.

[Eighth embodiment]

The light emitting device 1H (1) according to the eighth embodiment will be described. The light emitting device 1H according to the eighth embodiment includes the light source 2 and the wavelength converter 3 (3H). The light emitting device 1H according to the eighth embodiment is the light emitting device 1D according to the fourth embodiment from which the light guide body 20 is removed. The light emitting device 1H according to the eighth embodiment differs from the light emitting device 1D according to the fourth embodiment only in the light guide body 20. Thus, the action not including the light guide body 20 is mainly described below, and for other members, the description of their configurations and actions is omitted or simplified.

(Action)

The action of the light emitting device 1H will be described. First, the primary light 6 emitted by the light source 2 is emitted on the front 3a of the wavelength converter 3H. Most of the primary light 6 enters the wavelength converter 3H from the front 3a of the wavelength converter 3H, and the remainder is reflected by the front 3a. In the wavelength converter 3H, the second wavelength-converted light 9 is emitted by the second phosphor 8 excited by the primary light 6, and the first wavelength-converted light 7 is emitted by the first phosphor 4 excited by the primary light 6 and/or the second wavelength-converted light 9. Then, the first wavelength-converted light 7 and the second wavelength-converted light 9 are emitted from the front 3a. In this way, the output light 15 including the primary light 6, the first wavelength-converted light 7, and the second wavelength-converted light 9 is emitted from the front 3a of the wavelength converter 3H.

(Preferred use)

The "preferred use" of the light emitting device 1H is the same as that of the light emitting device 1A, and thus the description is omitted.

(Effect)

The first phosphor 4 included in the wavelength converter 3H of the light emitting device 1H emits the output light 15 including the first wavelength-converted light 7 having high ICG excitation efficiency. Thus, with the light emitting device 1H, it is possible to provide a light emitting device having high ICG excitation efficiency and to provide an electronic apparatus using the light emitting device.

Since the light guide body 20 is not included, the light emitting device 1H is inexpensive.

[Electronic apparatus]

Using the above-described light emitting device 1 (1A to 1H), an electronic apparatus according to an embodiment can be obtained. That is, the electronic apparatus according to an embodiment includes any one of the light emitting devices 1A to 1H (1) according to the embodiments.

As described above, the light emitting device 1 (1A to 1H) emits the output light 15 including the first wavelength-converted light 7 having high ICG excitation efficiency.

The light emitting device 1 according to an embodiment emits the output light 15 including the first wavelength-converted light 7 having high ICG excitation efficiency, and thus when the light emitting device 1 according to an embodiment is used for a medical device or a cosmetic device, the miniaturization of the medical device or the cosmetic device is easy and a large therapeutic effect is expected.

Thus, when the electronic apparatus according to an embodiment is a medical device or a cosmetic device, it is easy to miniaturize the medical device or the cosmetic device, and a large therapeutic effect is expected. When the electronic apparatus according to an embodiment is a medical device or a cosmetic device and the electronic apparatus according to an embodiment uses a fluorescent agent, such as ICG, the light emitting device 1 is preferable because the light emitting device 1 emits the output light 15 including the first wavelength-converted light 7 having high ICG excitation efficiency.

The light emitting device according to an embodiment emits the output light 15 including the first wavelength-converted light 7 having high ICG excitation efficiency, and thus when the light emitting device 1 according to an embodiment is used for a sensing device or a sensing system, the miniaturization of the sensing device or the sensing system is easy.

Thus, when the electronic apparatus according to an embodiment incudes the light emitting device according to an embodiment and a sensing device or a sensing system, the miniaturization of the sensing device or the sensing system is easy. An example of the sensing device is an endoscope. An example of the sensing system is an endoscope system.

When the electronic apparatus according to an embodiment includes the light emitting device according to an embodiment and a sensing device or a sensing system, it is preferable that the electronic apparatus according to an embodiment use a fluorescent agent, such as ICG. The reason why it is preferable is that the light emitting device 1 emits the output light 15 including the first wavelength-converted light 7 having high ICG excitation efficiency. The action of the electronic apparatus including the light emitting device according to an embodiment and a sensing device or a sensing system is such that a fluorescent agent is irradiated with the first wavelength-converted light 7 included in the output light 15 and the sensing device or a sensing system detects fluorescence emitted by the fluorescent agent. Thus, it is possible for the electronic apparatus including the light emitting device according to an embodiment and the sensing device or the sensing system to detect an observation object chemically coupled with ICG.

When the electronic apparatus according to an embodiment is a sensing device or a sensing system, specific examples include an endoscope and an endoscope system. An endoscope and an endoscope system are described below.

[Endoscope and endoscope system]

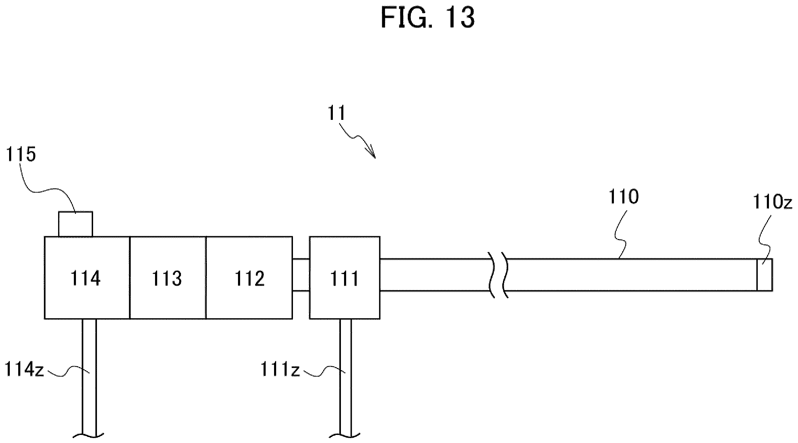

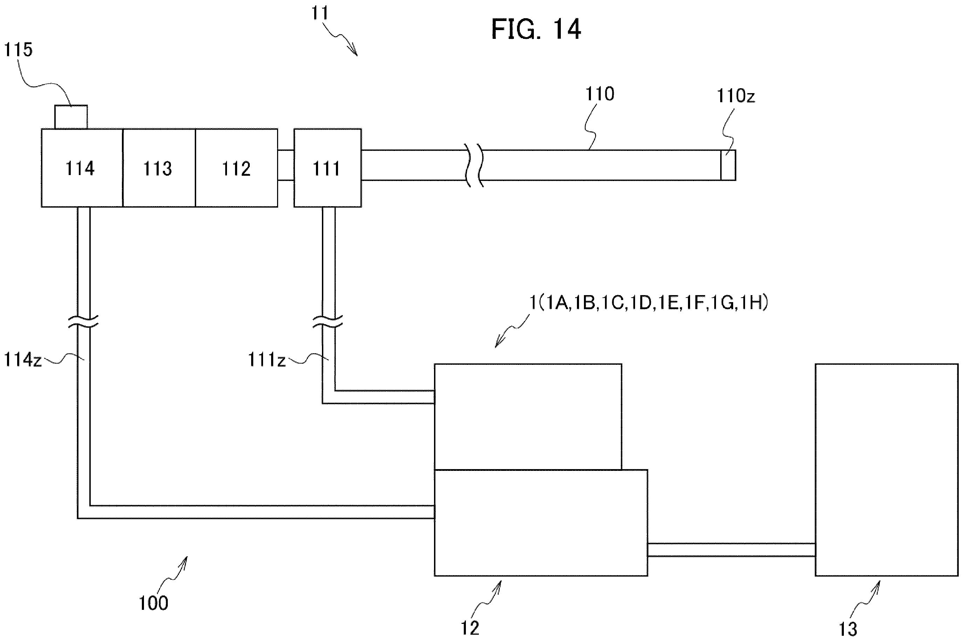

An endoscope according to an embodiment includes the above-described medical or cosmetic light emitting device. An example of the endoscope according to an embodiment and an example of an endoscope system using the endoscope are described below with reference to Figs. 13 and 14. Note that the description below is an example of an endoscope including the light emitting device 1 (IA to 1H) that emits visible light in addition to near-infrared light.

(Endoscope)

As illustrated in Fig. 13, an endoscope 11 includes a scope 110, a light source connector 111, a mount adapter 112, a relay lens 113, a camera head 114, and an operating switch 115.

The scope 110 is an elongated light guide member that can guide light from end to end and is inserted into a body when used. The scope 110 has an imaging window 110z at the tip, and an optical material, such as optical glass or optical plastic, is used for the imaging window 110z. The scope 110 further includes an optical fiber that guides light introduced from the light source connector 111 to the tip, and an optical fiber through which an optical image entering from the imaging window 110z is transmitted.

The mount adapter 112 is a member for attaching the scope 110 to the camera head 114. Various scopes 110 are detachably mounted on the mount adapter 112.

The light source connector 111 introduces illumination light, from the light emitting device 1, with which an affected part or the like in a body is irradiated. In this embodiment, the illumination light includes visible light and near-infrared light. The light introduced into the light source connector 111 is guided to the tip of the scope 110 through an optical fiber, and is emitted on an affected part or the like in a body through the imaging window 110z. Note that as illustrated in Fig. 13, the transmission cable 111z for guiding illumination light from the light emitting device 1 to the scope 110 is connected to the light source connector 111. The transmission cable 111z may include an optical fiber.

The relay lens 113 converges the optical image transmitted through the scope 110 on an imaging surface of the image sensor. Note that the relay lens 113 may be adjusted in focus and in magnification by moving the lens according to the amount of operation of the operation switch 115.

The camera head 114 includes a color separation prism inside. The color separation prism separates light converged by the relay lens 113 into four colors of R light (red light), G light (green light), B light (blue light), and IR light (near-infrared light). The color separation prism is made from a translucent member, such as glass.

The camera head 114 further includes an image sensor inside as a detector. For example, four image sensors are provided, and each of the four image sensors converts an optical image formed on each imaging surface into an electrical signal. The image sensor is not limited, but at least one of a CCD (charge coupled device) or a CMOS (complementary metal oxide semiconductor) is usable. The four image sensors are dedicated sensors for receiving IR component (near-infrared component) light, B component (blue component) light, R component (red component) light, and G component (green component) light.

The camera head 114 may have a color filter inside instead of the color separation prism. The color filter is provided on the imaging surface of the image sensor. For example, four color filters are provided, and the four color filters receive light converged by the relay lens 113 and selectively transmit R light (red light), G light (green light), B light (blue light), and IR light (near-infrared light).

A color filter that selectively transmits IR light preferably includes a barrier film that cuts off a reflection component of near-infrared light (IR light) included in the illumination light. This causes only fluorescence of IR light emitted by ICG to form an image on the imaging surface of the image sensor for IR light. This makes it easier to clearly observe the affected part that emits light due to ICG.

Note that as illustrated in Fig. 13, a signal cable 114z for transmitting an electrical signal from the image sensor to a CCU 12, which is described below, is connected to the camera head 114.

In the endoscope 11 having such a configuration, light from a subject is guided through the scope 110 to the relay lens 113 and then is transmitted through the color separation prism in the camera head 114 to form images on the four image sensors.

(Endoscope system)

As illustrated in Fig. 14, the endoscope system 100 includes the endoscope 11 for imaging the inside of a subject, the CCU (camera control unit) 12, the light emitting device 1, and a display device 13, such as a display.

The CCU 12 includes at least an RGB signal processing unit, an IR signal processing unit, and an output unit. The CCU 12 implements functions of the RGB signal processing unit, the IR signal processing unit, and the output unit by executing a program stored in an internal or external memory of the CCU 12.

The RGB signal processing unit converts electrical signals of the B component, the R component, and the G component sent from the image sensor into video signals that can be displayed on the display device 13 and outputs them to the output unit. The IR signal processing unit converts the electrical signal of the IR component sent from the image sensor, into a video signal and outputs it to the output unit.

The output unit outputs at least one of the video signals of the RGB color components or the video signal of the IR component to the display device 13. For example, the output unit outputs video signals based on either one of a simultaneous output mode or a superimposed output mode.

In the simultaneous output mode, the output unit simultaneously outputs the RGB image and the IR image on separate screens. The simultaneous output mode enables an affected part to be observed by comparing the RGB image with the IR image on the separate screens. In the superimposed output mode, the output unit outputs a composite image in which the RGB image and the IR image are superimposed. The superimposed output mode enables clear observation of an affected part emitting light due to ICG in the RGB image, for example.

The display device 13 displays an image of an object, such as an affected part, on a screen based on video signals sent from the CCU 12. In the simultaneous output mode, the display device 13 divides the screen into multiple screens and displays the RGB image and the IR image on each screen side by side. In the superimposed output mode, the display device 13 displays a composite image in which the RGB image and the IR image are superimposed on each other on a single screen.

(Action)

Next, the action of the endoscope 11 and the endoscope system 100 according to the embodiment will be described. When a subject is observed using the endoscope system 100, indocyanine green (ICG), which is a fluorescent substance, is first administered to the subject. This causes ICG to accumulate at a site (affected part) such as a lymph node or a tumor.

Then, visible light and near-infrared light are introduced from the light emitting device 1 to the light source connector 111 through the transmission cable 111z. The light introduced into the light source connector 111 is guided to the tip side of the scope 110 and projected from the imaging window 110z to illuminate the area surrounding an affected part, including the affected part. The light reflected at the affected part and the like, and fluorescence emitted by ICG are guided to the rear end of the scope 110 through the imaging window 110z and the optical fiber, converge at the relay lens 113, and enter the color separation prism inside the camera head 114.

In the color separation prism, of the incident light, IR component light separated by an IR separation prism is captured as an optical image of an infrared component by an image sensor for IR. B component light separated by a blue separation prism is captured as an optical image of a blue component by an image sensor for blue. R component light separated by a red separation prism is captured as an optical image of a red component by an image sensor for red. G component light separated by a green separation prism is captured as an optical image of a green component by an image sensor for green.

An IR component electrical signal converted by the image sensor for IR is converted into a video signal at the IR signal processing unit inside the CCU 12. B component, R component, and G component electrical signals converted by the image sensor for RGB are converted into each video signal by the RGB signal processing unit inside the CCU 12. The video signal of the IR component and the video signals of the B, R, and G components are synchronously output to the display device 13.

When the simultaneous output mode is set inside the CCU 12, the RGB image and the IR image are simultaneously displayed on two screens of the display device 13. When the superimposed output mode is set inside the CCU 12, a composite image in which the RGB image and the IR image are superimposed is displayed on the display device 13.

As described above, the endoscope 11 according to the embodiment includes the medical or cosmetic light emitting device 1. Thus, it is possible with the endoscope 11 to clearly observe an affected part by efficiently exciting a fluorescent agent to cause the fluorescent agent to emit light.

The endoscope 11 according to the embodiment preferably further includes a detector that detects fluorescence emitted by a fluorescent agent that has absorbed the first wavelength-converted light 7. When the endoscope 11 includes the detector that detects the fluorescence emitted by the fluorescent agent in addition to the light emitting device 1 as one body, it is possible to identify an affected part only with the endoscope. Thus, it is not necessary to open an abdomen wide to identify an affected part as in the past, and medical examination and treatment can be performed with less burden on the patient. Since a doctor using the endoscope 11 can accurately identify an affected part, it is possible to improve the efficiency of treatment.

EXAMPLES

The light emitting device according to the present embodiment is described in more detail below with reference to examples, but the present embodiment is not limited to these.

[Example 1]

(Preparation of phosphor)

An oxide phosphor was synthesized through a preparation procedure using a solid-state reaction. Specifically, an oxide phosphor expressed by the composition formula of $Gd_3(Ga_{0.97}Cr_{0.03})_2(GaO_4)_3$ was synthesized. Note that the following compound powders were used as main raw materials in synthesizing the oxide phosphor.

Gadolinium oxide ($Gd_2O_3$): purity 3N, manufactured by Kojundo Chemical Lab. Co., Ltd.

Gallium oxide ($Ga_2O_3$): purity 4N, manufactured by Nippon Rare Metal, Inc.

Chromium oxide ($Cr_2O_3$): purity 3N, manufactured by Kojundo Chemical Lab. Co., Ltd.

First, the above raw materials were weighed to give a compound $Gd_3(Ga_{0.97}Cr_{0.03})_2(GaO_4)_3$ of a stoichiometric composition. Then, dry mixing was performed using a mortar and a pestle, and a raw material for calcination was obtained.

The above-described calcination raw material was moved to an alumina crucible having a lid and was calcined for 2 hours in air at 1600 °C using a box-type electric furnace, and then the calcined material was lightly cracked to obtain a phosphor of an example 1. Note that the sample after calcination was confirmed to be $Gd_3(Ga_{0.97}Cr_{0.03})_2(GaO_4)_3$ using an X-ray diffraction method.

(Evaluation)

<Evaluation of fluorescence spectrum>

The fluorescence spectrum of the phosphor was evaluated using an absolute PL quantum yield measuring device C9920-02G (manufactured by Hamamatsu Photonics K.K.). Fig. 10 illustrates a fluorescence spectrum $L_{GGG}$ when excited at an excitation wavelength of 450 nm.

<Evaluation of emission peak wavelength $\lambda_{MAX}$>

Based on the fluorescence spectrum of the phosphor, an emission peak wavelength $\lambda_{MAX}$ was measured, which is a peak wavelength of a fluorescence intensity maximum value peak indicating the fluorescence intensity maximum value in the fluorescence spectrum. The result is shown in Table 1.

<Evaluation of fluorescence spectrum half-width>

From the fluorescence spectrum of the phosphor, the fluorescence spectrum half-width was measured. The result is shown in Table 1.

< Evaluation of 800-nm fluorescence intensity percentage $L_{800nm}$>

The 800-nm fluorescence intensity percentage Lsoonm was calculated, which is the percentage of an emission intensity at the wavelength of 800 nm with respect to an emission peak intensity (fluorescence intensity maximum value) at the fluorescence intensity maximum value peak of the fluorescence spectrum. The result is shown in Table 1.

<Evaluation of ICG excitation efficiency>

The ICG excitation efficiency was calculated using the following equation (AI) and the "fluorescence spectrum $L_{GGG}$" measured in <Evaluation of fluorescence spectrum> described above.
[Math 2]

$$\int_{500nm}^{800nm} [\text{GSG emission spectrum intensity}] \times [\text{ICG absorption spectrum intensity}]$$

$$= [\text{ICG excitation efficiency}] \quad (A1)$$

Specifically, the ICG excitation efficiency was calculated by substituting the "intensity of the fluorescence spectrum $L_{GGG}$" (= GGG emission spectrum intensity) into the "GSG emission spectrum intensity" in equation (AI) above. The result is shown in Table 1.

[Table 1]

| | Composition | $\lambda_{MAX}$ (nm) | Fluorescence spectrum half-width (nm) | $L_{800nm}$ (%) | ICG excitation efficiency (a.u.) | Remarks |
|---|---|---|---|---|---|---|
| Example 1 | $Gd_3(Ga_{0.97}Cr_{0.03})_2(GaO_4)_3$ | 735 | 92 | 45 | 52 | GGG-based phosphor |
| Example 2 | $(Gd_{0.8}La_{0.2})_3(Ga_{0.97}Cr_{0.03})_2(GaO_4)_3$ | 745 | 91 | 48 | 53 | GLGG-based phosphor |
| Comparative example 1 | $(Gd_{0.75}La_{0.25})_3(Ga_{0.5}Sc_{0.47}Cr_{0.03})_2(GaO_4)_3$ | 765 | 109 | 81 | 47 | GSG-based phosphor |

[Example 2]

(Preparation of phosphor)

An oxide phosphor was synthesized through a preparation procedure using a solid-state reaction. Specifically, an oxide phosphor expressed by the composition formula of $(Gd_{0.8}La_{0.2})_3(Ga_{0.97}Cr_{0.03})_2(GaO_4)_3$ was synthesized. Note that the following compound powders were used as main raw materials in synthesizing the oxide phosphor.

Gadolinium oxide $(Gd_2O_3)$: purity 3N, manufactured by Kojundo Chemical Lab. Co., Ltd.

As an La raw material, lanthanum oxide $(La_2O_3)$: purity 3N, manufactured by Kojundo Chemical Lab. Co., Ltd., or lanthanum hydroxide $(La(OH)_3)$: purity 3N, manufactured by Shin-Etsu Chemical Co., Ltd.

Gallium oxide $(Ga_2O_3)$: purity 4N, manufactured by Nippon Rare Metal, Inc.

Chromium oxide $(Cr_2O_3)$: purity 3N, manufactured by Kojundo Chemical Lab. Co., Ltd.

First, the above raw materials were weighed to give a compound $(Gd_{0.8}La_{0.2})_3(Ga_{0.97}Cr_{0.03})_2(GaO_4)_3$ of a stoichiometric composition. Then, dry mixing was performed using a mortar and a pestle, and a raw material for calcination was obtained.

The above-described calcination raw material was moved to an alumina crucible having a lid, calcined for 2 hours in air at 1400 °C using a box-type electric furnace, and then the calcined material was lightly cracked to obtain a phosphor of an example 2. Note that the sample after calcination was confirmed to be $(Gd_{0.8}La_{0.2})_3(Ga_{0.97}Cr_{0.03})_2(GaO_4))_3$ using an X-ray diffraction method.

(Evaluation)

As in the example 1, the fluorescence spectrum $(L_{GLGG})$ of the phosphor, the emission peak wavelength $\lambda_{MAX}$, the fluorescence spectrum half-width, the 800-nm fluorescence intensity percentage $L_{800nm}$, and the ICG excitation efficiency were evaluated. The results are shown in Fig. 10 and Table 1.

Note that the ICG excitation efficiency was calculated by substituting the "intensity of the fluorescence spectrum $L_{GLGG}$ (= GLGG emission spectrum intensity)" into the "GSG emission spectrum intensity" in equation (A1) above.

[Comparative example 1]

(Preparation of phosphor)

An oxide phosphor was synthesized through a preparation procedure using a solid-state reaction. Specifically, an oxide phosphor expressed by the composition formula of $(Gd_{0.75}La_{0.25})_3(Ga_{0.5}Sc_{0.47}Cr_{0.03})_2(GaO_4)_3$ was synthesized. The following compound powders were used as main raw materials in synthesizing the oxide phosphor.

Gadolinium oxide $(Gd_2O_3)$: purity 3N, manufactured by Kojundo Chemical Lab. Co., Ltd.

As an La raw material, lanthanum oxide $(La_2O_3)$: purity 3N, manufactured by Kojundo Chemical Lab. Co., Ltd., or lanthanum hydroxide $(La(OH)_3)$: purity 3N, manufactured by Shin-Etsu Chemical Co., Ltd.

Gallium oxide $(Ga_2O_3)$: purity 4N, manufactured by Nippon Rare Metal, Inc.

Scandium oxide $(Sc_2O_3)$: Purity 4N, manufactured by Shin-Etsu Chemical Co., Ltd.

Chromium oxide $(Cr_2O_3)$: purity 3N, manufactured by Kojundo Chemical Lab. Co., Ltd.

First, the above raw materials were weighed to give a compound $(Gd_{0.75}La_{0.25})_3(Ga_{0.5}Sc_{0.47}Cr_{0.03})_2(GaO_4)_3$ of a stoichiometric composition. Then, dry mixing was performed using a mortar and a pestle, and a raw material for calcination was obtained.

The above-described calcination raw material was moved to an alumina crucible having a lid, calcined for 2 hours in air at 1400 °C using a box-type electric furnace, and then the calcined material was lightly cracked to obtain a phosphor of a comparative example 1. Note that the sample after calcination was confirmed to be $(Gd_{0.75}La_{0.25})_3(Ga_{0.5}Sc_{0.47}Cr_{0.03})_2(GaO_4)_3$ using an X-ray diffraction method.

(Evaluation)

As in the example 1, the fluorescence spectrum $(L_{GSG})$ of the phosphor, the emission peak wavelength $\lambda_{MAX}$, the fluorescence spectrum half-width, the 800-nm fluorescence intensity percentage $L_{800nm}$, and the ICG excitation efficiency were evaluated. The results are shown in Fig. 10 and Table 1.

Note that the ICG excitation efficiency was calculated by substituting the "intensity of the fluorescence spectrum Lose" into the "GSG emission spectrum intensity" in equation (A1) above.

The entire contents of Japanese Patent Application No. 2020-108503 (filed on June 24, 2020) are incorporated herein by reference.

Although the contents of the present embodiment have been described above along with the examples, it is obvious to those skilled in the art that the present embodiment is not limited to these descriptions and can be modified and improved in various ways.

INDUSTRIAL APPLICABILITY

The present disclosure is capable of providing a light emitting device having a high ICG excitation efficiency and an electronic apparatus using the light emitting device.

REFERENCE SIGNS LIST

| | |
|---|---|
| \1, 1A, 1B, 1C, 1D, 1E, 1F, 1G, 1H | Light emitting device |
| 2 | Solid-state light emitting element (light source) |
| 3, 3A, 3B, 3C, 3D, 3E, 3F, 3G, 3H | Wavelength converter |
| 4 | First phosphor |
| 6 | Primary light |
| 7 | First wavelength-converted light |
| 8 | Second phosphor |
| 9 | Second wavelength-converted light |
| 11 | Endoscope |
| 15 | Output light |
| 13 | Display device |
| 20 | Light guide body |
| 31 | Broad fluorescent component |
| 41 | Host crystal |
| 100 | Endoscope system |
| 110 | Scope (light guide member) |
| 110z | Imaging window |
| 111 | Light source connector |

**Claims**

1. A light emitting device, comprising:

    a light source configured to emit primary light; and
    a wavelength converter that includes a first phosphor configured to absorb the primary light and emit first wavelength-converted light, wherein
    the light emitting device configures to emit output light including the first wavelength-converted light,
    the first wavelength-converted light is near-infrared light having a fluorescence intensity maximum value within a wavelength range of 700 nm or more and less than 800 nm,
    the first wavelength-converted light mainly contains a broad fluorescent component based on an electron energy transition of $^4T_2 \rightarrow {}^4A_2$ of $Cr^{3+}$, and
    the broad fluorescent component has a fluorescence spectrum half-width that is less than 100 nm.

2. The light emitting device according to claim 1, wherein
    a percentage of a fluorescence intensity at a wavelength of 800 nm with respect to the fluorescence intensity maximum value in a fluorescence spectrum of the first wavelength-converted light is less than 60%.

3. The light emitting device according to claim 1 or 2, wherein
    in the first phosphor, a host crystal includes a garnet crystal structure represented by a general formula shown below:

$$A'_{3}B'_{2}(C'O_4)_3 \qquad (1).$$

4. The light emitting device according to claim 3, wherein
    the B' contains one element having an ion radius of 0.5 Å or more and less than 0.8 Å.

**5.** The light emitting device according to claim 4, wherein
the B' is Al, Ga, or Sc.

**6.** The light emitting device according to any one of claims 1 to 5, wherein

the light source is a solid-state light emitting element, and
the primary light is at least one of blue light having a maximum intensity of a spectral distribution within a wavelength range of 400 nm or more and less than 500 nm, or red light having a maximum intensity of a spectral distribution within a wavelength range of 600 nm or more and less than 660 nm.

**7.** The light emitting device according to any one of claims 1 to 6, wherein
the light source and the wavelength converter are arranged spaced apart.

**8.** The light emitting device according to any one of claims 1 to 7, further comprising:

a light guide body arranged between the light source and the wavelength converter, the light guide body configured to guide the primary light to the wavelength converter, wherein
the primary light passes through the inside of the light guide body.

**9.** The light emitting device according to any one of claims 1 to 8, wherein
the primary light is laser light.

**10.** The light emitting device according to any one of claims 1 to 9, wherein
the light emitting device is a medical or cosmetic light emitting device.

**11.** An electronic apparatus, comprising:
the light emitting device according to any one of claims 1 to 10.

**12.** The electronic apparatus according to claim 11, comprising:

the light emitting device; and
a sensing device or a sensing system.

**13.** The electronic apparatus according to claim 12, wherein

the electronic apparatus uses a fluorescent agent, and
the sensing device or the sensing system detects fluorescence emitted by the fluorescent agent through irradiation of the fluorescent agent with the first wavelength-converted light contained in the output light.

FIG. 1

FIG. 2

FIG. 3

FIG. 4

FIG. 5

FIG. 6

FIG. 7

FIG. 8

## FIG. 9

## FIG. 10

## FIG. 11

FIG. 12

EP 4 174 967 A1

# FIG. 13

FIG. 14

## INTERNATIONAL SEARCH REPORT

| International application No. |
| --- |
| PCT/JP2021/021910 |

**A. CLASSIFICATION OF SUBJECT MATTER**

H01L 33/50(2010.01)i; A61B 1/00(2006.01)i; A61N 5/06(2006.01)i; A61N
5/067(2006.01)i; C09K 11/80(2006.01)i; H01L 33/00(2010.01)i; H01S
5/0225(2021.01)i; H01S 5/02251(2021.01)i; H01S 5/02255(2021.01) i
FI: H01L33/50; H01L33/00 L; H01S5/0225; H01S5/02251; H01S5/02255;
C09K11/80; A61B1/00 511; A61N5/06 Z; A61N5/067

According to International Patent Classification (IPC) or to both national classification and IPC

**B. FIELDS SEARCHED**

Minimum documentation searched (classification system followed by classification symbols)
H01L33/00-33/64; H01S5/00-5/50; A61B1/00-1/32; C09K11/00-11/89; A61N5/00-
5/10

Documentation searched other than minimum documentation to the extent that such documents are included in the fields searched
| | |
| --- | --- |
| Published examined utility model applications of Japan | 1922-1996 |
| Published unexamined utility model applications of Japan | 1971-2021 |
| Registered utility model specifications of Japan | 1996-2021 |
| Published registered utility model applications of Japan | 1994-2021 |

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
| --- | --- | --- |
| Y | WO 2018/230207 A1 (PANASONIC IP MANAGEMENT CO., LTD.) 20 December 2018 (2018-12-20) paragraphs [0035]-[0070], fig. 1-6 | 1-13 |
| Y | CN 110964523 A (UNIV JIANGSU NORMAL) 07 April 2020 (2020-04-07) paragraphs [0002]-[0004], [0014]-[0018], [0042]-[0048], fig. 2, 6-9 | 1-13 |
| Y | CN 111218279 A (UNIV BEIJING SCIENCE & TECH) 02 June 2020 (2020-06-02) page 3, line 6 to page 3, line 8 | 1-13 |
| Y | JP 2015-93 A (OLYMPUS CORP.) 05 January 2015 (2015-01-05) paragraphs [0020]-[0054], fig. 2-4 | 12-13 |

| ☐ | Further documents are listed in the continuation of Box C. | ☒ | See patent family annex. |
| --- | --- | --- | --- |

| * | Special categories of cited documents: |
| --- | --- |
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
| --- | --- |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
| --- | --- |
| 18 August 2021 (18.08.2021) | 31 August 2021 (31.08.2021) |

| Name and mailing address of the ISA/ Japan Patent Office 3-4-3, Kasumigaseki, Chiyoda-ku, Tokyo 100-8915, Japan | Authorized officer Telephone No. |
| --- | --- |

Form PCT/ISA/210 (second sheet) (January 2015)

**INTERNATIONAL SEARCH REPORT**

Information on patent family members

International application No.

PCT/JP2021/021910

| Patent Documents referred in the Report | Publication Date | Patent Family | Publication Date |
|---|---|---|---|
| WO 2018/230207 A1 | 20 Dec. 2018 | US 2020/0109331 A1 paragraphs [0045]-[0080], fig. 1-6 EP 3640206 A1 CN 110730762 A | |
| CN 110964523 A | 07 Apr. 2020 | (Family: none) | |
| CN 111218279 A | 02 Jun. 2020 | (Family: none) | |
| JP 2015-93 A | 05 Jan. 2015 | US 2014/0371527 A1 paragraphs [0038]-[0072], fig. 2-4 | |

Form PCT/ISA/210 (patent family annex) (January 2015)

**EP 4 174 967 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 5812461 B
- JP 2018518046 A

- JP 2020108503 A

37